(19)

Europäisches
Patentamt
European
Patent Office
Office européen
des brevets

(11) **EP 2 728 013 A1**

(12) **EUROPEAN PATENT APPLICATION**

(43) Date of publication:
**07.05.2014 Bulletin 2014/19**

(51) Int Cl.:
***C12Q 1/68*** (2006.01)

(21) Application number: **12190754.7**

(22) Date of filing: **31.10.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Universitätsspital Basel
4031 Basel (CH)**

(72) Inventors:
- **Kinter, Jochen
  4103 Bottmingen (CH)**
- **Sinnreich, Michael
  4051 Basel (CH)**

(74) Representative: **Schmauder & Partner AG
Patent- & Markenanwälte VSP
Zwängiweg 7
8038 Zürich (CH)**

(54) **Method for the simultaneous amplification of a plurality of different nucleic acid target sequences**

(57)   The present invention relates to a method for the simultaneous amplification of a plurality of different nucleic acid target sequences comprising the steps of providing a plurality of different nucleic acid polymers as templates, each template comprising a specific target sequence and a primer annealing sequence located downstream of the target sequence, and
amplifying the template by a polymerase dependent amplification reaction using a primer oligonucleotide comprising a primer sequence which is at least essentially complementary to the primer annealing sequence.

The method is characterized in that for the polymerase dependent amplification reaction a set of primer oligonucleotides is used, said set comprising at least two primer oligonucleotides which are able to anneal to the primer annealing sequence of the same template and which differ from each other in the efficiency for the polymerase dependent amplification reaction to take place.

Fig. 1A

EP 2 728 013 A1

## Description

FIELD OF THE INVENTION

The present invention relates to a method for the simultaneous amplification of a plurality of different nucleic acid target sequences, to a kit for carrying out the method and to a library of nucleic acid polymers, in particular a DNA or a RNA library. The invention further relates to the use of the method for a gene probe assay as well as in molecular cloning.

BACKGROUND OF THE INVENTION

The detection of specific nucleic acid polymers is an important tool for diagnostic medicine and molecular biology research. Gene probe assays currently play a role e.g. in identifying infectious organisms such as bacteria and viruses, in probing the expression of normal genes and in identifying mutant genes such as oncogenes, in tissue typing for compatibility preceding tissue transplantation, in matching tissue or blood samples for forensic medicine, and for exploring homology among genes from different species.

Ideally, a gene probe assay should be sensitive, specific and easily automatable. The requirement for sensitivity (i.e. low detection limits) has been greatly improved by the development of the polymerase chain reaction (PCR) and other amplification technologies which allow researchers to amplify exponentially a specific target sequence before analysis. The PCR technology is for example described in US-B-4,683,202.

In the last years progress has been made by the development of new technologies which are promising in reducing costs and accelerating the development of new molecular diagnostics. DNA analysis instruments are becoming increasingly more powerful in the capacity of sequence analysis. DNA resequencing microarrays (Chee et al., 1996, Patil et al., 2001) and high throughput parallel sequencing instruments (Margulies et al., 2005, Shendure et al., 2005) are currently used for whole genome analyses of low complexity genomes down to single nucleotide resolution. However, the human genome remains too large to access without complexity reduction by directed amplification of specific sequences. To match the throughput of these instruments, the amplification bottleneck needs to be addressed with more efficient technologies. Enrichment of target sequences becomes therefore key for comprehensive resequencing of human exons at a fraction of the cost of whole-genome sequencing. Recently several sequence capture methods have been developed like molecular inversion probe technology (Dahl et al., 2007, Dahl et al., 2005, Porreca et al., 2007), approaches using microarray technologies (Okou et al., 2007, Hodges et al., 2007, Albert et al., 2007), hybridization in solution technologies using RNA oligo capture probes (Gnirke et al., 2009), or microfluidic technology using emulsion PCR in small droplets(Tewhey et al., 2009).

In theory, the currently most powerful and fastest amplification technology is PCR and is widely used in molecular diagnostics. To increase assay throughput and allow for more efficient use of precious DNA samples, simultaneous amplification of several targets can be carried out by combining many specific primer pairs in individual PCRs (Chamberlain et al., 1988, Shigemori et al., 2005). However, it is one of the crucial problems with PCR that when large numbers of specific primer pairs are added to the same reaction, both correct and incorrect amplicons are generated. In addition, even when primer dimers can be avoided and specific amplification is achieved the targets have different PCR efficiencies due to amplicon length and sequence properties (GC content). At a later stage, this skews the uniformity of the products to the point where many amplicons drop out in favor of highly efficient amplified amplicons and artifacts. In order to optimize multiplex PCR, the concentrations of primer, buffer dNTPs, enzymes, and $MgCl_2$ need to be determined empirically for each set of primer combinations. This is a time-consuming process which needs to be conducted for each lot of the produced assay. A successful multiplex PCR is not guaranteed even after exhaustive optimization experiments.

Even with careful attention paid to the design of primers in case of multiplexing, PCR is usually limited to 10-20 simultaneous reactions before yield and evenness is compromised by the accumulation of irrelevant amplification products (Syvänen, 2005, Broude et al., 2001). Therefore, large numbers of separate PCRs are typically performed whenever many genomic sequences need to be analyzed. Thus the major challenge in multiplexing PCR is to overcome two major problems: the incompatibility of primers leading to unspecific amplifications (like primer dimers) and the differences in amplification efficiencies of different targets.

Considering the drawbacks of the methods according to the state of the art, the problem to be solved by the present invention is thus to provide a simple, rapid and inexpensive method for simultaneously amplifying a plurality of different nucleic acid target sequences, in particular DNA and/or RNA target sequences. In this regard, the method shall allow amplification of practically all target sequences at a more uniform abundance than with conventional methods, and in particular with standard PCR. This invention provides a novel multiplex technology solving both fundamental problems thereby allowing uniform amplification of multiple targets in one single reaction.

## SUMMARY OF THE INVENTION

*Principle of methodology*

**Efficiency tag PCR**

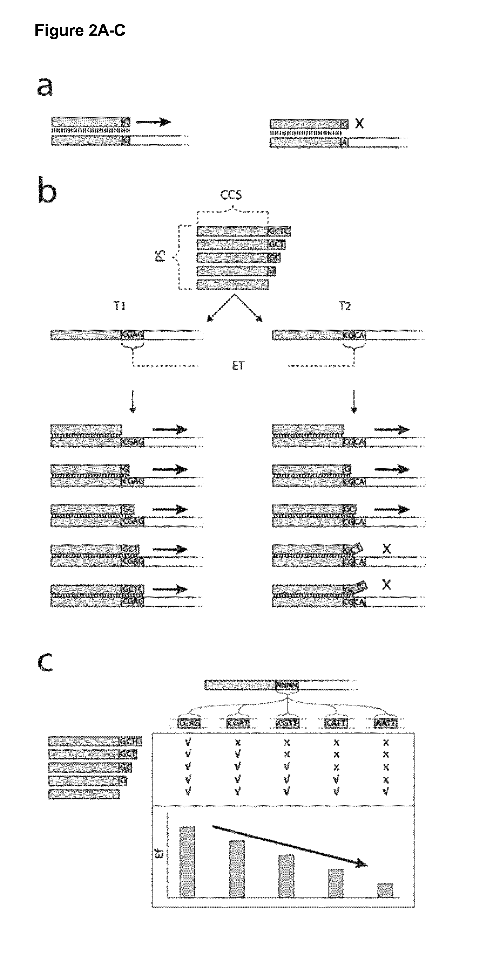

The principle of this method is based on the fact that a single mismatch at the 3 prime end of the primer/template hybrid strongly inhibits PCR amplification. Although a single 3 prime mismatch may allow primer annealing, the extension step performed by the polymerase is inhibited (Figure 2a). The Efficiency Tag PCR (etPCR) takes advantage of this fact to regulate the PCR efficiency of each single target. Instead of using one single primer pair etPCR uses two sets of primers, each set consisting of similar primers which have a common core sequence allowing the annealing to the target but which differ in length, leading to differences in the 3 prime end (Figure 2b). In such an etPCR reaction where a template has a perfect match to the entire complementary sequence, all primers can be used by the polymerase for synthesizing a new strand. This results in an amplification with efficiencies corresponding to normal PCR. In case a mismatch is introduced in the 3 prime part of the template's priming site, all primers will still be able to bind, however only some primers will allow extension by the polymerase. Therefore the portion of primers participating in the amplification reaction will depend on the numbers of mismatches at the 3 prime priming site of the target. Thus the efficiency of the PCR can be regulated by the introduction of specific mismatches into the template. In case of a set of 5 primers, there is the possibility to tune the efficiency in 5 different gradations by introducing up to 4 mismatches (Figure 2C). The number of different efficiency levels which can be used is determined by the size of the tag (Levels = Length of tag + 1). Introduction of two tags, one at each side of the template, multiplies the possibilities of different gradations (Levels = [length of forward tag +1] x [length of reverse tag + 1]). Using an Efficiency Tag of 4 bases at both ends of a template will allow efficiency adjustment in 25 different nuances. This efficiency tag PCR brings new opportunities for multitemplate amplification. The adjustment of the PCR efficiency of each single template by using the proper tags will lead to a uniform amplification of all templates. In addition the use of a universal pair of primer set with a common sequence for all templates eliminates the problem of primer dimers. EtPCR only requires a library consisting of templates, flanked with the efficiency tag and the common priming sequence.

**Sequence capture: Preparation of efficiency tagged template library from genomic DNA (preferred embodiment)**

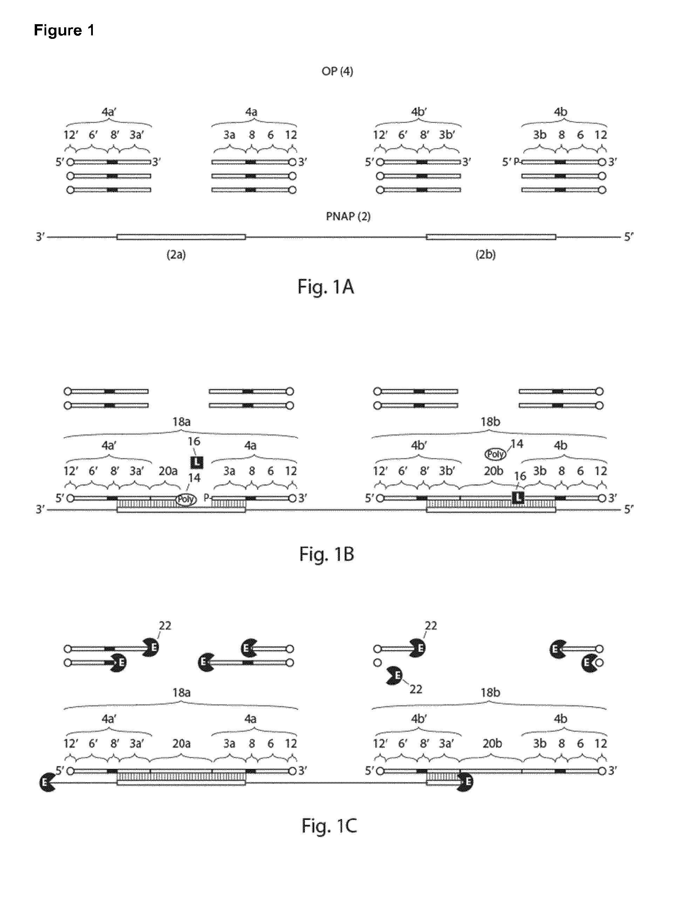

To select the regions of interest (i.e. certain exons of a gene) from a DNA source like genomic DNA or cDNA, we developed a novel sequence capture approach. The method results in single stranded copy of the regions of interest flanked by efficiency tags and the common priming sequence for further uniform amplification with etPCR as described above. Targeting of specific sequences is achieved through a hybridization step of oligonucleotides flanking the region of interest. The oligonucleotides consist of four different parts: a target specific sequence, an efficiency tag, the common priming sequence and a "exonuclease block" consisting of phosphothioates at their outer end (Figure 1). After hybridization with the flanking oligos, the gap consisting of the region of interest will be filled by a polymerase reaction. The nick between the synthesized strand and the oligo will be closed by a ligation reaction. Prior to subsequent amplification, the unbound oligos will be removed through digestion with exonucleases. Newly synthesized DNA fragments of the targeted region will be protected from exonuclease digestion, as this region will be flanked by phosphorothioates on either side, acting as "exonuclease blocks". Individual oligonucleotides, however, will be digested by added exonucleases, since they harbor only one exonuclease block on one of their ends. This results in a newly synthesized single DNA strand consisting of the desired genomic region flanked by efficiency tag and universal priming sequences which can be used for etPCR. This sequence capture in conjunction with the novel etPCR technology allows uniform multiplex amplification from any source of DNA by eliminating primer incompatibility and nonuniform amplification, the two fundamental problems of multiplex PCR.

The present invention relates to the following embodiments (1) to (15).

(1) A method for the simultaneous amplification of a plurality of different nucleic acid target sequences comprising the steps of

providing a set of forward primer oligonucleotides capable of annealing to the same nucleotide sequence, said set comprising a first forward primer oligonucleotide having the structure

5'- X-N$^1$ -3',

and a second forward primer oligonucleotide having the structure

5'- X-N$^1$-N$^2$ -3',

wherein X is a nucleotide sequence which is capable of annealing to a first primer annealing sequence X', N$^1$ is nothing or consists of one or more nucleotides, and N$^2$ consists of one or more nucleotides;

providing a plurality of different nucleic acid polymers as templates, each template comprising (i) a forward primer annealing sequence X' which is complementary to the nucleotide sequence X, and (ii) a specific target sequence; and amplifying the templates by a polymerase dependent amplification reaction using said set of forward primer oligonucleotides and one or more reverse primer oligonucleotide(s), characterized in that the 3'-terminal nucleotide of the first forward primer oligonucleotide, when annealed to the templates, has a perfect match with at least two different template sequences, and the 3'-terminal nucleotide of the second forward primer oligonucleotide, when annealed to the templates, has a mismatch with at least one of said at least two different template sequences and a perfect match with at least one of said at least two different template sequences.

(2) The method of (1), further comprising the steps of providing a set of reverse primer oligonucleotides capable of annealing to the same nucleotide sequence, comprising a first reverse primer oligonucleotide having the structure
5'- Y-$M^1$ -3',
and a second reverse primer oligonucleotide having the structure
5'- Y-$M^1$-$M^2$ -3',
wherein Y is a nucleotide sequence which is capable of annealing to a reverse primer annealing sequence, $M^1$ is nothing or consists of one or more nucleotides, and $M^2$ consists of one or more nucleotides;
wherein each template further comprises a reverse primer annealing sequence which is complementary to the nucleotide sequence Y, the target sequence is located between the forward primer annealing sequence and the reverse primer annealing sequence, and the polymerase dependent amplification reaction is carried out using said set of forward primer oligonucleotides and said set of reverse primer oligonucleotides,
characterized in that the 3'-terminal nucleotide of the first reverse primer oligonucleotide, when annealed to the templates, has a perfect match with at least two different template sequences, and the 3'-terminal nucleotide of the second reverse primer oligonucleotide, when annealed to the templates, has a mismatch with at least one of said at least two different template sequences and a perfect match with at least one of said at least two different template sequences

(3) The method of (2), wherein the number of templates is v, and each template comprises the structure
5'- X - $et^{Xw}$ - $T^w$ - $et^{Y'w}$ - Y' -3'
wherein
v is an integer greater than 1,
w is an integer running from 1 to v, each specific template being assigned an individual value w,
X is as defined in claim 1,
$et^{Xw}$ is a first efficiency tag sequence,
$T^w$ is the target sequence or complement thereof,
$et^{Y'w}$ is the complementary sequence of a second efficiency tag sequence,
Y' is the reverse primer annealing sequence.

(4) The method of (3), characterized in that each efficiency tag sequence comprises from 1 to 10 nucleotides, preferably from 2 to 7 nucleotides, most preferably from 3 to 5 nucleotides.

(5) The method of (3) or (4), characterized in that each of the templates is provided by the subsequent steps of:

providing a single stranded primal nucleic acid polymer comprising a primal target sequence to be amplified;

hybridizing to the 5'-end of the primal target sequence an oligonucleotide probe, the sequence of the oligonucleotide probe comprising a portion of the target sequence complementary to the 5'-end of the primal target sequence, the primer annealing sequence and the efficiency tag sequence, and to the 3'-end of the primal target sequence a further oligonucleotide probe, the sequence of the further oligonucleotide probe comprising a portion of the target sequence complementary to the 3'-end of the primal target sequence, the primer annealing complementary sequence and the efficiency tag complementary sequence;

synthesizing a strand complementary to the primal target sequence by means of a polymerase and a ligase to produce the template; and

isolating the templates produced.

(6) The method of (5), characterized in that the ends of the template produced are protected against exonucleases.

(7) The method of (5) or (6), characterized in that the template produced comprises free ends, one or more nucleotides in the region of both ends being modified to form an exonuclease protection.

(8) The method of (7), characterized in that the one or more modified nucleotides are phosphorothioated.

(9) The method of any of (5) to (8), characterized in that the step of isolating the templates produced is performed by digesting the remaining nucleic acid components with an exonuclease.

(10) A library of nucleic acid polymers, in particular a DNA or a RNA library, comprising a plurality of templates as defined in any one of (2) to (9).

(11) A kit for carrying out the method according to any of (1) to (9) comprising
a first set of oligonucleotide probes, the sequence of each oligonucleotide probe of the first set comprising
a portion of a target sequence complementary to the 5'-end of a primal target sequence to be amplified,
an efficiency tag sequence, and
a primer annealing sequence,
a second set of oligonucleotide probes, the sequence of each oligonucleotide probe of the second set comprising
a primer annealing complementary sequence,
an efficiency tag complementary sequence, and
a portion of the target sequence complementary to the 3'-end of the primal target sequence,
a first set of different primer oligonucleotides comprising a primer sequence which is at least essentially complementary to the primer annealing sequence of the oligonucleotide of the first set and differing from each other in the
length of their extension downstream of the primer sequence, and
a second set of different primer oligonucleotides comprising a primer sequence which is at least essentially complementary to the further primer annealing sequence obtainable by synthesizing a strand complementary to the
template comprising the primer annealing complementary sequence, the primer oligonucleotides of the second set
differing from each other in the length of their extension downstream of the primer sequence.

(12) The kit of (11) further comprising a polymerase and a ligase.

(13) The use of the method according to any of (1) to (9) for a gene probe assay, in particular for identifying infectious
organisms or mutant genes.

(14) The use of the method according to any of (1) to (9) in molecular cloning.

DETAILED DESCRIPTION

The method of the invention comprises the step of providing a set of forward primer oligonucleotides. The set comprises r different forward primer oligonucleotides, wherein r is an integer greater than 1. That is, r is at least 2, preferably at least 3, more preferably at least 4, most preferably at least 5. Typically, r ranges from 2 to 20, preferably from 2 to 10, more preferably from 3 to 7, most preferably r is 4 or 5.
A first forward primer oligonucleotide has the structure
5'- X-$N^1$ -3',
and a second forward primer oligonucleotide having the structure
5'- X-$N^1$-$N^2$ -3',
wherein X is a nucleotide sequence which is capable of annealing to a first primer annealing sequence X', $N^1$ is nothing or consists of one or more nucleotides, and $N^2$ consists of one or more nucleotides. Each forward primer within the set of forward primer oligonucleotides is capable of annealing to the same nucleotide sequence via its portion X. The sequence $N^1$ may be nothing or consist of one or more nucleotides, e.g. of 1 to 20 nucleotides. The sequence $N^2$ may independently consist of 1 to 20 nucleotides. Preferably, $N^2$ consists of 1 to 10, more preferably of 1 to 5, most preferably of 1 to 3 nucleotides, e.g. of 1, 2 or 3 nucleotides. Preferably, $N^2$ consists of one nucleotide.
The different forward primer oligonucleotides typically differ only in their 3' ends, i.e. in the sequence which is located 3' to the sequence X.
In one aspect of the invention, the set of forward primers comprises r different forward primer oligonucleotides, and the structure of primer No. q is 5'- X-$(n)_{(q-1)}$ -3', wherein q ranges from 1 to r, r is as defined above, and each n independently is any nucleotide. In other words, the first forward primer oligonucleotide (i.e. q=1) has the structure: 5'- X -3'; the second forward primer oligonucleotide (i.e. q=2) has the structure: 5'- X-n -3'; the third forward primer oligonucleotide (i.e. q=3) has the structure: 5'- X-nn -3'; the fourth forward primer oligonucleotide (i.e. q=4) has the structure: 5'- X-nnn -3'; and the fifth forward primer oligonucleotide (i.e. q=5) has the structure: 5'- X-nnnn -3'. This list can be extended. Preferably, each n is independently selected from the group consisting of the nucleotides a, c, g and t.

According to a preferred embodiment of this aspect, the set of forward primer oligonucleotides comprises 4 different forward primer oligonucleotides (r=4), the first forward primer oligonucleotide has the structure: 5'- X -3'; the second forward primer oligonucleotide has the structure: 5'- X-n -3'; the third forward primer oligonucleotide has the structure: 5'- X-nn -3'; and the fourth forward primer oligonucleotide has the structure: 5'- X-nnn -3'.

According to a another preferred embodiment of this aspect, the set of forward primer oligonucleotides comprises 5 different forward primer oligonucleotides (r=5), the first forward primer oligonucleotide has the structure: 5'- X -3'; the second forward primer oligonucleotide has the structure: 5'- X-n -3'; the third forward primer oligonucleotide has the structure: 5'- X-nn -3'; the fourth forward primer oligonucleotide has the structure: 5'- X-nnn -3'; and the fifth forward primer oligonucleotide has the structure: 5'- X-nnnn -3'.

According to a yet another preferred embodiment of this aspect, the set of forward primer oligonucleotides comprises 6 different forward primer oligonucleotides (r=6), the first forward primer oligonucleotide has the structure: 5'- X -3'; the second forward primer oligonucleotide has the structure: 5'- X-n -3'; the third forward primer oligonucleotide has the structure: 5'- X-nn -3'; the fourth forward primer oligonucleotide has the structure: 5'- X-nnn -3'; the fifth forward primer oligonucleotide has the structure: 5'- X-nnnn -3'; and the sixth forward primer oligonucleotide has the structure: 5'- X-nnnnn -3'.

X is a nucleotide sequence which is capable of annealing to a first primer annealing sequence. X has a length of at least 6 nucleotides, preferably of at least 8, more preferably of at least 10, most preferably of at least 12 nucleotides. Typically, the length of X ranges from 6 to 100, preferably from 8 to 75, more preferably from 10 to 50, more preferably from 12 to 30, most preferably from 15 to 25 nucleotides.

The method of the invention further comprises providing a plurality of different nucleic acid polymers as templates, each template comprising a specific target sequence and a forward primer annealing sequence which is complementary to the nucleotide sequence X. The number of different nucleic acid templates is at least 2, preferably at least 3, more preferably at least 5. Typically, the number of different templates provided ranges from 2 to 100,000, preferably from 3 to 1000, more preferably from 4 to 500, more preferably from 5 to 200, most preferably from 10 to 50. Preferably, the forward primer annealing sequence is located upstream to the specific target sequence, i.e. 5' to the target sequence. It is preferred that the forward primer annealing sequence and the target sequence are separated by a so-called 'efficiency tag sequence' as explained further below.

The length of the target sequence may range from about 10 to about 50,000 nucleotides; preferably it ranges from about 50 to about 10,000 nucleotides, more preferably from about 75 to about 5,000 nucleotides, most preferably from about 100 to about 1,500 nucleotides. The templates usually have identical primer annealing sequences and differ in their target sequences.

The method of the invention further comprises amplifying the templates by a polymerase dependent amplification reaction using said set of forward primer oligonucleotides and one or more reverse primer oligonucleotide(s). In one embodiment, the reverse primer is a single oligonucleotide capable of annealing to substantially all template molecules, preferably at a location downstream to the target sequence. In another embodiment, a set of reverse primer oligonucleotides is used. This latter embodiment will be explained further below.

According to this invention the 3'-terminal nucleotide of the first forward primer oligonucleotide has a perfect match with at least two different template sequences, whereas the second forward primer oligonucleotide has a mismatch with at least one of said at least two different template sequences. That is, in its simplest variant, the first forward primer oligonucleotide will amplify two different templates, and the second forward primer oligonucleotide will amplify only one of these two different templates.

The method of the invention may further comprise the steps of providing a set of reverse primer oligonucleotides capable of annealing to the same nucleotide sequence within the template sequence. The set comprises p different reverse primer oligonucleotides, wherein p is an integer greater than 1. That is, p is at least 2, preferably at least 3, more preferably at least 4, most preferably at least 5. Typically, p ranges from 2 to 20, preferably from 2 to 10, more preferably from 3 to 7, most preferably p is 4 or 5. Each reverse primer within the set of reverse primer oligonucleotides is capable of annealing to the same nucleotide sequence via its portion Y. The first reverse primer oligonucleotide has the structure 5'- Y-$M^1$ -3',

and a second reverse primer oligonucleotide having the structure

5'- Y-$M^1$-$M^2$ -3',

wherein Y is a nucleotide sequence which is capable of annealing to a reverse primer annealing sequence, $M^1$ is nothing or consists of one or more nucleotides, and $M^2$ consists of one or more nucleotides. The sequence $M^1$ may be nothing or consist of one or more nucleotides, e.g of 1 to 20 nucleotides. The sequence $M^2$ may independently consist of 1 to 20 nucleotides. Preferably, $M^2$ consists of 1 to 10, more preferably of 1 to 5, most preferably of 1 to 3 nucleotides, e.g. of 1, 2 or 3 nucleotides. Preferably, $M^2$ consists of one nucleotide.

The different reverse primer oligonucleotides typically differ only in their 3' ends, i.e. in the sequence which is located 3' to the sequence Y.

In one aspect of the invention, the set of reverse primers comprises p different reverse primer oligonucleotides, and the structure of reverse primer No. s is 5'- Y-(n)$_{(s-1)}$ -3', wherein s ranges from 1 to p, p is as defined above, and each n independently is any nucleotide. In other words, the first reverse primer oligonucleotide (i.e. s=1) has the structure: 5'- Y -3'; the second reverse primer oligonucleotide (i.e. s=2) has the structure: 5'- Y-n -3'; the third reverse primer oligonucleotide (i.e. s=3) has the structure: 5'- Y-nn -3'; the fourth reverse primer oligonucleotide (i.e. s=4) has the structure: 5'- Y-nnn -3'; and the fifth reverse primer oligonucleotide (i.e. s=5) has the structure: 5'- Y-nnnn -3'. This list can be extended. Preferably, each n is independently selected from the group consisting of the nucleotides a, c, g and t.

According to a preferred embodiment of this aspect, the set of reverse primer oligonucleotides comprises 4 different reverse primer oligonucleotides (p=4), the first reverse primer oligonucleotide has the structure: 5'- Y -3'; the second reverse primer oligonucleotide has the structure: 5'- Y-n -3'; the third reverse primer oligonucleotide has the structure: 5'- Y-nn -3'; and the fourth reverse primer oligonucleotide has the structure: 5'- Y-nnn -3'.

According to a another preferred embodiment of this aspect, the set of reverse primer oligonucleotides comprises 5 different reverse primer oligonucleotides (p=5), the first reverse primer oligonucleotide has the structure: 5'- Y -3'; the second reverse primer oligonucleotide has the structure: 5'- Y-n -3'; the third reverse primer oligonucleotide has the structure: 5'- Y-nn -3'; the fourth reverse primer oligonucleotide has the structure: 5'- Y-nnn -3'; and the fifth reverse primer oligonucleotide has the structure: 5'- Y-nnnn -3'.

According to a yet another preferred embodiment of this aspect, the set of reverse primer oligonucleotides comprises 6 different reverse primer oligonucleotides (p=6), the first reverse primer oligonucleotide has the structure: 5'- Y -3'; the second reverse primer oligonucleotide has the structure: 5'- Y-n -3'; the third reverse primer oligonucleotide has the structure: 5'- Y-nn -3'; the fourth reverse primer oligonucleotide has the structure: 5'- Y-nnn -3'; the fifth reverse primer oligonucleotide has the structure: 5'- Y-nnnn -3'; and the sixth reverse primer oligonucleotide has the structure: 5'- Y-nnnnn -3'.

Y is a nucleotide sequence which is capable of annealing to a reverse primer annealing sequence. Y has a length of at least 6 nucleotides, preferably of at least 8, more preferably of at least 10, most preferably of at least 12 nucleotides. Typically, the length of Y ranges from 6 to 100, preferably from 8 to 75, more preferably from 10 to 50, more preferably from 12 to 30, most preferably from 15 to 25 nucleotides.

According to this invention the 3'-terminal nucleotide of the first reverse primer oligonucleotide has a perfect match with at least two different template sequences, whereas the second reverse primer oligonucleotide has a mismatch with at least one of said at least two different template sequences. That is, in its simplest variant, the first reverse primer oligonucleotide will amplify two different templates, and the second reverse primer oligonucleotide will amplify only one of these two different templates.

In one embodiment, each template comprises a reverse primer annealing sequence which is complementary to the nucleotide sequence Y, the target sequence is located between the forward primer annealing sequence and the reverse primer annealing sequence, and the polymerase dependent amplification reaction is carried out using said set of forward primer oligonucleotides and said set of reverse primer oligonucleotides.

In one aspect of this invention, the number of templates is v, and each template comprises the structure

5'- X - et$^{Xw}$ - T$^w$ - et$^{Y'w}$ - Y' -3'

wherein

v is an integer greater than 1,

w is an integer running from 1 to v, each specific template being assigned an individual value w,

X is as defined in claim 1,

et$^{Xw}$ is a first efficiency tag sequence,

T$^w$ is the target sequence or complement thereof,

et$^{Y'w}$ is the complementary sequence of a second efficiency tag sequence,

Y' is the reverse primer annealing sequence.

As will be shown in detail below, the present invention allows a "graded" amplification reaction to be performed in the sense that the amplification efficiency can be adapted specifically for each target sequence. In particular in multiplex PCR, the amplification efficiency of the different targets can thus be levelled leading to a more or less uniform number of replicates for each target.

According to a very straightforward and thus particularly preferred embodiment, each template comprises between the primer annealing sequence and the target sequence a specific efficiency tag sequence (ETS). Depending on their specific ETS, the templates can thus be divided into different template groups, whereby the number of primer oligonucleotides having an extension fully matching the ETS or fully matching a portion of the ETS, is different from template group to template group. The ETS thus permits on the one hand a selective polymerase-mediated extension for primer oligonucleotides having an extension fully matching the ETS or fully matching a portion of the ETS. On the other hand, only inefficient polymerase mediated extension will occur for primer oligonucleotides having an extension that does not match or only partly matches the ETS or a portion thereof.

By appropriately attributing the different ETS to the different targets, a less efficient amplification can be achieved for high abundance amplicons.

More specifically, in a "graded" amplification reaction the lowest grade of efficiency is achieved for an ETS which shows no complementarity with any of the extensions of the primer oligonucleotides, since for this, the only primer oligonucleotide of the set that can be extended by polymerase is the one having no extension at all. A higher grade is achieved for an ETS showing complementarity with the first nucleotide of the extension of the oligonucleotides, since for this, the primer oligonucleotide having no extension at all and the primer oligonucleotide that is extended by one single nucleotide will anneal and can be extended by polymerase. An even higher efficiency is achieved for an ETS showing complementarity with the first two nucleotides of the extension and so on.

The ETS preferably comprises from 1 to 10 nucleotides, more preferably from 2 to 7 nucleotides, most preferably from 3 to 5 nucleotides. If, for example, an ETS having 4 nucleotides is used, five different efficiency grades can be established, one for an ETS fully corresponding to all nucleotides of the extension of the primer oligonucleotide, one for an ETS complementary only to the first three nucleotides of the extension, one for an ETS complementary only to the first two nucleotides of the extension, one for an ETS complementary only to the first nucleotide of the extension and one for an ETS which shows no complementarity with the extension at all.

According to a particularly preferred embodiment, the primer annealing sequence is identical for all templates. Thus, primer oligonucleotides comprising a universal primer sequence can be used in this embodiment, allowing both amplification of targets and their subsequent sequencing.

As for the set of primer oligonucleotides described above, the further primer annealing sequence is preferably identical for all templates. Thus, primer oligonucleotides comprising a universal primer sequence can also be used for the further set used in this embodiment.

It is in this regard also preferred that the only difference between the primer oligonucleotides of the further set is in the length of their extension, as it is the case for the set of primer oligonucleotides described above. This allows the template complementary strands to be divided into different "complementary strand groups", whereby the number of primer oligonucleotides of the further set having an extension fully matching the further ETS or a portion thereof, is different from "complementary strand group" to "complementary strand group". The ETS thus permits a selective polymerase mediated extension for extended primer oligonucleotides having an extension fully matching the further ETS or fully matching a portion of the ETS, which on the one hand allows for selective and thus highly efficient amplification of low efficient amplifiable targets, and an insufficient annealing of all other primer oligonucleotides, which on the other hand allows for less efficient amplification of high abundance targets, as mentioned above in connection with the ETS of the templates.

According to a further preferred embodiment, one or more regions of at least a portion of the templates and/or of the primer oligonucleotides encode a bar code, thus allowing attributing the replicated templates to their origins in an easy manner. In particular when DNA from different patients is assayed in parallel, such as in multiplex PCR, the bar code allows attributing the replicated DNA sequences to each individual patient (Binladen et al. (2007) PLoS ONE 2(2):e197, incorporated herein by reference).

For providing the templates comprising - in addition to the specific target sequence - also an ETS and a primer annealing sequence, a method is preferably used comprising the subsequent steps of:

providing a single stranded primal nucleic acid polymer comprising a primal target sequence to be amplified;

hybridizing to the 5'-end of the primal target sequence an oligonucleotide probe, the sequence of the oligonucleotide probe comprising a portion of the target sequence complementary to the 5'-end of the primal target sequence, the ETS and the primer annealing sequence, and to the 3'-end of the primal target sequence a further oligonucleotide probe, the sequence of the further oligonucleotide probe comprising a portion of the target sequence complementary to the 3'-end of the primal target sequence, the efficiency tag complementary sequence and the primer annealing complementary sequence;

synthesizing a strand complementary to the primal target sequence by means of a polymerase and a ligase to produce the template; and

isolating the templates produced.

In order to allow the ligase to close the nick between the strand produced and the oligonucleotide probe at the 3'-end (comprising the portion of the target complementary to the 5'-end of the primal target sequence), said oligonucleotide probe is generally 5'-end phosphorylated.

Thus, a newly synthesized single nucleic acid strand comprising the target sequence and the primer annealing sequences can be obtained which can then be used for amplification in a universal PCR.

Also, a "tailor-made" ETS can be introduced for each template by this method, ultimately allowing the amplification efficiency of each template to be modulated, as described in detail above.

Since the present invention is preferably used for gene probe assays, in particular for identifying infectious organisms or mutant genes, or for molecular cloning, the primal nucleic acid polymer is at least one selected from the group consisting of genomic DNA, mitochondrial DNA, mRNA, viral DNA, bacterial DNA, viral RNA and cDNA.

In order to allow easy isolation of the template produced, its ends are preferably protected against exonucleases. Particularly, the template produced comprises free ends, one or more nucleotides in the region of both ends being modified to form an exonuclease protection. More particularly, the one or more modified nucleotides are phosphorothioated.

Thus, the step of isolating the templates produced can be easily performed by digesting the remaining nucleic acid components using an exonuclease, leaving only the protected templates intact. The method for providing the templates is in the context of the present invention also referred to as "enrichment step".

According to a further preferred embodiment, the oligonucleotide probe is synthesized on a microchip.

Alternatively to the method using an ETS, it is also thinkable that a set of primer oligonucleotides is used at least some of which are blocked and thus not able to be extended by polymerases. Depending on the desired grade of efficiency for each template to be amplified, the ratio of blocked species to unblocked species can be adapted for each primer oligonucleotide. In view of achieving a more uniform amplification, the ratio of blocked primer oligonucleotides is higher for more abundant target sequences, and lower for less abundant target sequences.

According to a further aspect, the present invention further relates to a DNA or a RNA library comprising templates as described above. For example a plurality of multiple DNA probes, which are used for hybridization procedures, can be synthesized on a microchip and released as DNA probe pool in solution. Such a DNA probe pool can be amplified using PCR. Using universal primer annealing sequences on the synthesized DNA probes, the DNA probe pool can be amplified using one primer pair. The efficiency and the final amount of the single DNA probes mainly depend on the target sequence like length and sequence composition. The "graded" PCR can be applied to obtain a more uniform amplification and therefore nearly equal amounts of each DNA probe. In some instances it is desired to produce higher amount of certain DNA probes and/or lower amount of certain DNA probes. Using graded PCR the amplification efficiency of each DNA probe can be adjusted according the desired final probe amount.

According to a still further aspect, the present invention further relates to a kit for carrying out the method described above.

Said kit comprises

a first set of oligonucleotide probes, the sequence of each oligonucleotide probe of the first set comprising

- a portion of a target sequence complementary to the 5'-end of a primal target sequence to be amplified,

- an efficiency tag sequence, and

- a primer annealing sequence,

a second set of oligonucleotide probes, the sequence of each oligonucleotide probe of the second set comprising

- a primer annealing complementary sequence,

- an efficiency tag complementary sequence, and

- a portion of the target sequence complementary to the 3'-end of the primal target sequence,

a first set of different primer oligonucleotides comprising a primer sequence which is at least essentially complementary to the primer annealing sequence of the oligonucleotide of the first set and differing from each other in the length of their extension downstream of the primer sequence, and

a second set of different primer oligonucleotides comprising a primer sequence which is at least essentially complementary to the further primer annealing sequence obtainable by synthesizing a strand complementary to the template comprising the primer annealing complementary sequence, the primer oligonucleotides of the second set differing from each other in the length of their extension downstream of the primer sequence.

Preferably, the kit further comprises a polymerase and a ligase. As mentioned above, each oligonucleotide probe of the first set is typically 5'-end phosphorylated in order to allow the ligase to close the nick between said oligonucleotide probe and the strand produced. Further the first one to six nucleotides at the 3'-end of the first set of oligonucleotide probes are modified to be resistant against exonuclease cleavage. The last one to six nucleotides at the 5'-end of the second set of oligonucleotide probes are modified to be resistant against exonuclease cleavage. More particularly, the one or more modified nucleotides are phosphorothioated

Since the present invention is particularly suitable for gene probe assays, in particular for identifying infectious organisms or mutant genes, the present invention further relates to the use of the method described above for this purpose.

Alternatively, the present invention also relates to the use of the described method in molecular cloning.

The method present invention is illustrated further by way of the attached **Figures.**

**Fig. 1A-C** show schematically different steps of a method for providing templates as used in a preferred embodiment of the method according to the present invention. According to the method shown in Fig. 1 (that is the "enrichment step"), oligonucleotide probes are added to genomic DNA as primal nucleic acid polymer comprising one or more primal target sequences.

In the embodiment shown in Fig. 1, the primal nucleic acid polymer (PNAP) 2 comprises two primal target sequences 2a, 2b (see Fig. 1A).

The oligonucleotide probes (OP) 4 can be divided into two parts:

Each of the oligonucleotides of the first part 4a, 4b comprises a portion 3a, 3b, respectively, of a target sequence, complementary to the 5'-end of one of the primal target sequences 2a, 2b, respectively, a primer annealing sequence 6 and an ETS 8 located between the portion of a target sequence and the primer annealing sequence.

Each of the oligonucleotide probes of the second part 4a', 4b' comprises a portion 3a', 3b', respectively, of a target sequence complementary to the 3'-end of one of the primal primal target sequences 2a, 2b, respectively, a primer annealing complementary sequence 6' and an efficiency tag complementary sequence 8' located between the portion of the target sequence and the primer annealing complementary sequence.

Further, the oligonucleotide probes of the first part comprise an exonuclease-block 12 at their 3'-end, whereas the oligonucleotide probes of the second part comprise an exonuclease-block 12' at their 5'-end. The exonuclease-block can be achieved in numerous ways. According to a preferred embodiment, phosphorothioated, nuclease resistant nucleotides are added to both ends of the flanked target sequence.

Then, the oligonucleotide probes 4a, 4b of the first part are hybridized with the 5'-end of the respective primal target sequence 2a, 2b and the oligonucleotide probes 4a', 4b' of the second part are hybridized with the 3'-end of the respective primal target sequence 2a, 2b. Hybridisation comprises both denaturation of the genomic DNA, typically carried out at 95°C for 10 minutes, and annealing of the oligonucleotide probes, typically at about 60°C for 14 hours.

After hybridization, the gap between the flanking oligonucleotide probes is filled by synthesizing the strand complementary to the target sequence by means of a polymerase 14, which fills the gap by adding nucleotides. By means of a ligase 16, the nick between the strand produced and the probe at the 3'-end is ultimately closed, as shown in Fig. 1B. Incubation for filling the gap and closing the nick is typically at 60°C for about 24 hours.

By the synthesizing steps, templates 18a, 18b are achieved which comprise at their 3'-end a primer annealing sequence 6 followed by an ETS 8 and at their 5'-end a primer annealing complementary sequence 6' followed in direction to the 3'-end by an efficiency tag complementary sequence 8'. The target sequence 20a, 20b complementary to the primal target sequence 2a, 2b, respectively, is arranged between the ETS 8 and the efficiency tag complementary sequence 8'. Both the 3'- and the 5'-end of the template are protected by an exonuclease block 12, 12', respectively.

In a further step, an exonuclease or a mixture of multiple exonucleases 22 is added which digests all nucleic acid polymers that are not exonuclease-blocked at both ends, i.e. all nucleic acid polymers apart from the templates 18a, 18b produced, as shown in Fig. 1C.

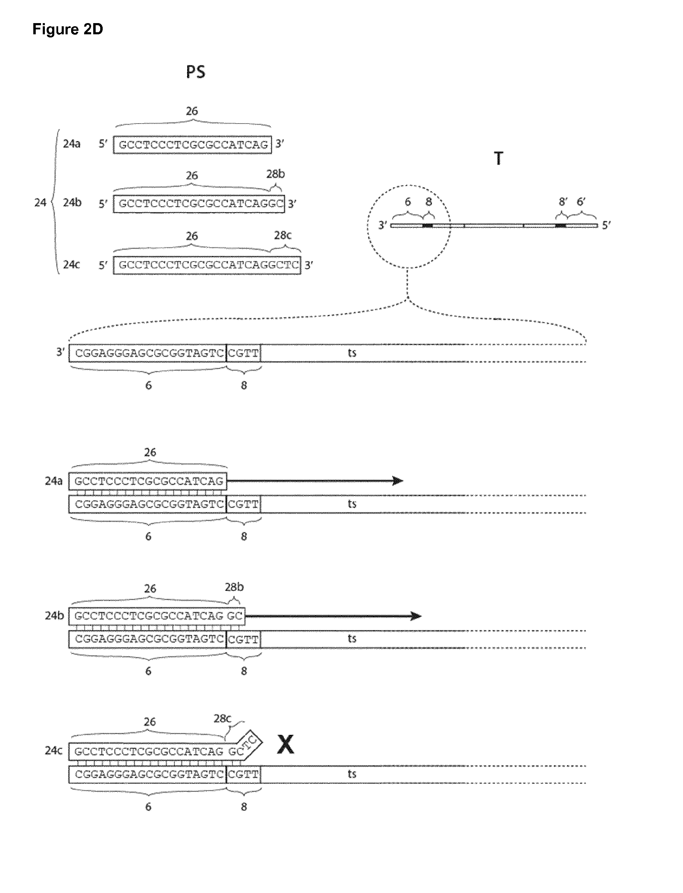

Based on the templates produced, PCR is then performed using a set of primer oligonucleotides 24. In Fig. 2D, three primer oligonucleotides 24a, 24b, 24c are shown. Said primer oligonucleotides 24a, 24b, 24c comprise a primer sequence 26, which in the embodiment shown is universal to all primer oligonucleotides of the set. Two of the three primer oligonucleotides shown further comprise an extension 28b, 28c downstream of the primer sequence 26 (see infra).

Abbreviations: E = Exonuclease; L = Ligase; Poly = Polymerase; PNAP = nucleic acid polymer; OP = oligonucleotide probes. **Fig. 2A-C** depicts the principle of the novel sequence capture technology. (a) To target specific genomic regions a left target oligonucleotide (LTO) and a right target oligonucleotide (RTO) are designed for each target elongation by the DNA polymerase is sensitive to mismatches at the 3 prime end of the primer. The presence of a single mismatch at the 3 prime end of the primer template hybrid is able to strongly reduce or totally inhibit PCR amplification. Therefore, the amplification process can be inhibited by the introduction of a mismatch into the primer binding sites of the template. (b) The novel PCR amplification method is able to specifically regulate the amplification efficiency of each single template of a template pool with common primer binding sites. Instead of a single common primer on each site a set of similar primer is used. The primers cover the identical sequence and just differ in length leading to different degrees of 3 prime extension. Template pools are designed that the shortest primer consisting of the common core sequence matches to all of the templates. Templates with perfect matches to all primers of the set are amplified without any efficiency reduction (T1). Introduction of mismatches within the efficiency tag of the template leads to a reduction of the amplification efficiency (T2). (c) By manipulating the degree of mismatches within the efficiency tag (ET) of the template the amplification efficiency can be regulated for each single template.Abbreviations: CCS = common core sequence; PS = primer set; T1 = target 1 with perfect matchas to all primers; T1 = target 2 with mismatches to certain primers; ET = efficiency tag; Ef = PCR Efficiency.

**Fig. 2D** shows schematically the annealing of three different primer oligonucleotides of one set to a given template. In the embodiment shown in Fig. 2D, the only difference between the primer oligonucleotides 24a, 24b, 24c of the set is in the length of their extension. Depending on the specific ETS attributed to a given target sequence, different numbers of primer oligonucleotides will allow polymerase dependent extension during the amplification step.

In the specific example shown in Fig. 2D, where the ETS 8 comprises four nucleotides, the last two nucleotides of the ETS are not complementary to the last two nucleotides of the primer oligonucleotide's extension in full length. Thus, only the primer oligonucleotides having a two-nucleotide extension (i.e. primer oligonucleotide 24b), a one-nucleotide extension (not shown) or no extension at all (i.e. primer oligonucleotide 24a) allows efficient polymerase dependent extension, whereas the primer oligonucleotides comprising a three-nucleotide extension (not shown) or four-nucleotide extension (i.e. primer oligonucleotide 24c) does not.

Depending on the specific ETS attributed to a given target sequence, the templates can be attributed to different template groups, the number of primer oligonucleotides having an extension fully matching the ETS or fully matching a portion of the ETS is different from template group to template group.

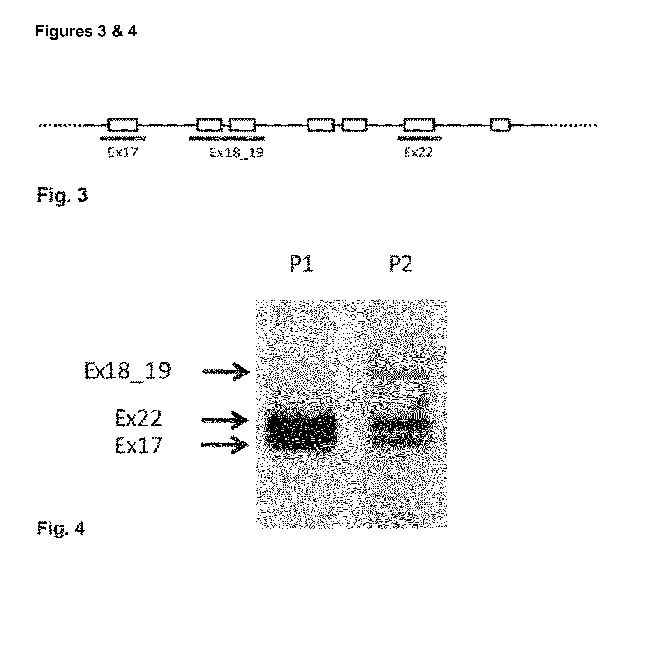

Abbreviations: PS = primer oligonucleotide set; T = targt; ts = target sequence.**Fig. 3** shows schematically the location of different exons of the calpain 3 gene targeted in a example 1 of the present invention discussed below.

Abbreviations: Ex17 = Exon 17; Ex18_19 = Exon 18 and exon 19; Ex22 = Exon 22.**Fig. 4** is a picture of an agarose gel subjected to agarose gel electrophoresis used for separating the nucleic acid target sequences amplified as described in example 1.

Abbreviations: P1 = standard PCR; P2 = efficiency tag PCR; Ex17 = Exon 17; Ex18_19 = Exon 18 and exon 19; Ex22 = Exon 22.

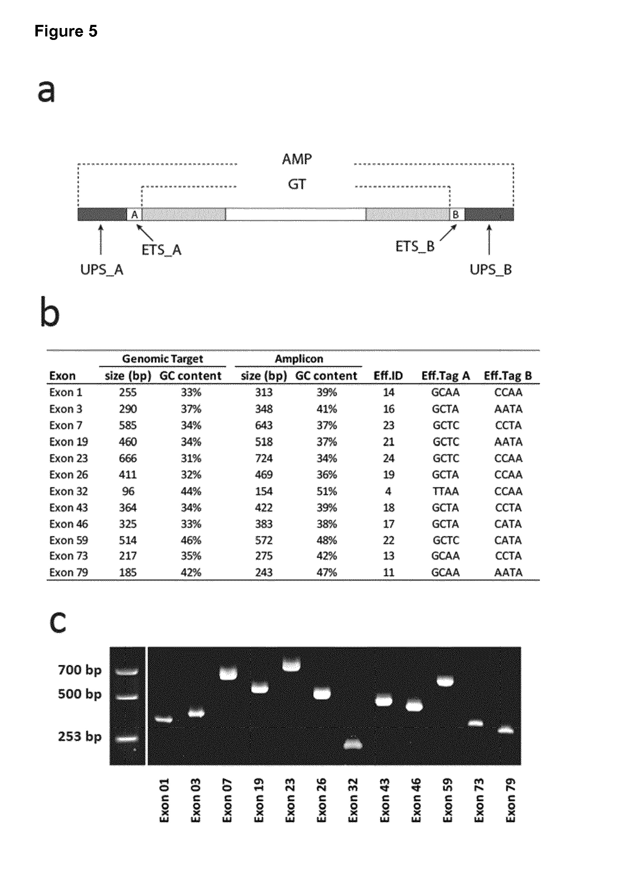

**Fig. 5** depicts different templates with efficiency tags and universal primer sequences. a) Templates with different genomic target sequences were generated for performing etPCR having universal primer sequences and efficiency tags at both ends. b) The table shows the different properties of the target sequence as well as the properties of the whole amplicon. Different efficiency tags were incorporated to analyze their performance in etPCR. C) Prior to etPCR analysis the different templates were verified by gel electrophoresis and purified.

Abbreviations: AMP = amplicon; GT = genomic target; ETS_A = efficiency tag sequence A; ETS_B = efficiency tag sequence B; UPS_A = universal priming site A; UPS_B = universal priming site B.

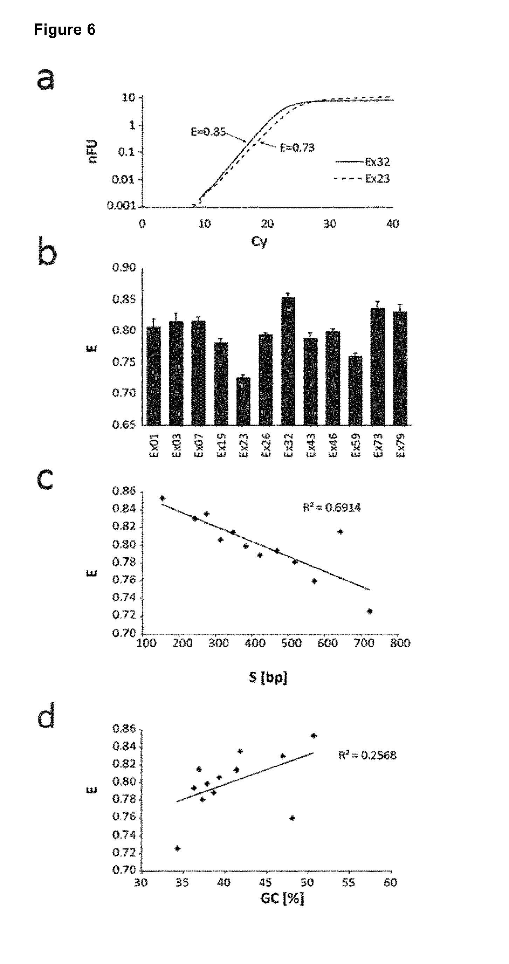

**Fig. 6** depicts variations in PCR Efficiency due to intrinsic properties. a) The different templates with the common primer site were used in standard qPCR using the same primer pair. The PCR efficiencies were measured by analyzing the exponential phase with the LinReg software. B) Significant differences in PCR efficiency could be detected between several templates. C) In our set of templates the intrinsic PCR efficiency strongly correlates with the length of the amplicons and show no correlation with the GC content (d).

Abbreviations: nFU = normalized Fluorescence Units; Cy = Cylces; S = amplicon size in base pairs; GC = GC content in percentage.

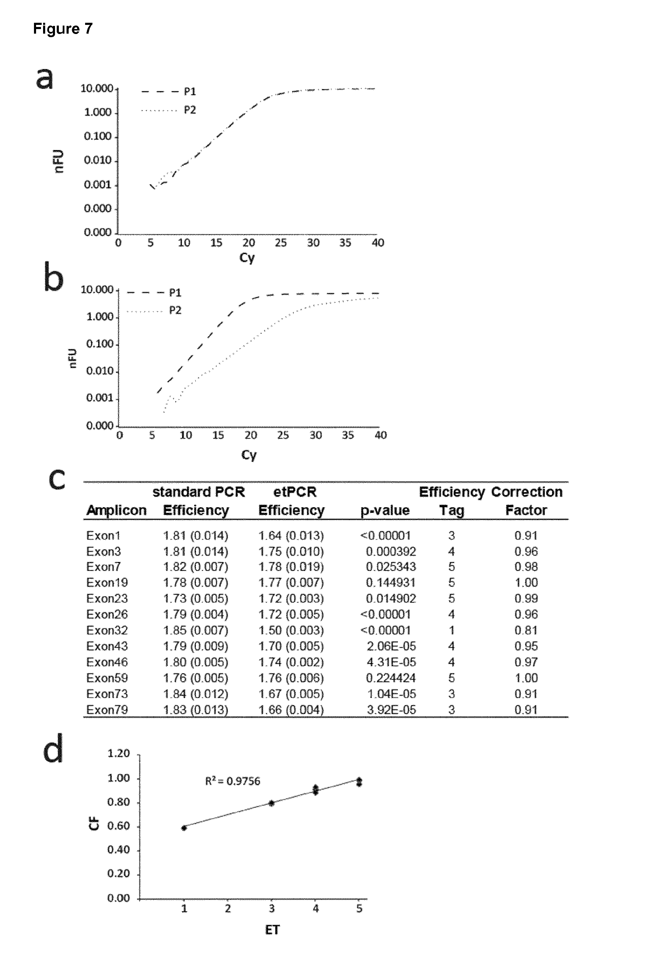

**Fig. 7** shows that etPCR can specifically modulate PCR efficiency. Efficiency Tag PCR was performed with the 12 template and compared with standard PCR. a) There was no difference observed with templates having no mismatches within the efficiency tag (Tag 5). b) By introducing mismatches into the tag less primer can participate in the PCR reaction resulting in a reduced PCR efficiency. c) All the tags harboring mismatches show significant reduced efficiencies and therefore allow specific manipulation of the amplification. The degree of reduction is defined by the correction factor shown in the table. d) Different templates with the same efficiency tag show similar correction factors. This allows the defined regulation of specific templates by the selection of certain tags. Abbreviations: nFU = normalized Fluorescence Units; Cy = Cylces; P1 = standard PCR; P2 = efficiency tag PCR; CF = correction factor; ET = efficiency tag.

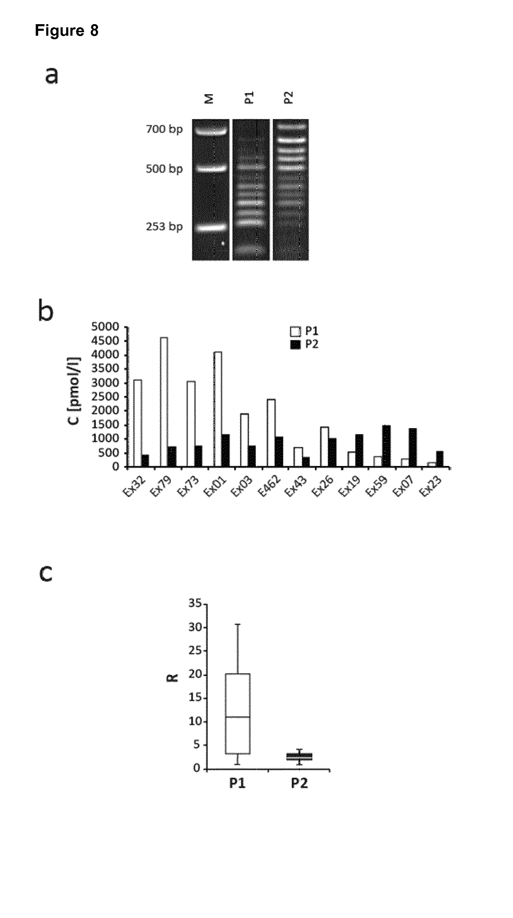

**Fig. 8** shows that etPCR can regulate PCR efficiency in multiplex reactions to produce uniform amplification. After a sequence capture reaction using 100 ng genomic DNA as described in Material and Methods either standard PCR or etPCR was performed. Using standard PCR the amplification of small templates was most efficient, whereas larger amplicons were hardly to detect on gel electrophoresis. When using etPCR large amplicons were easily detected and the observed pattern resembled a more uniform amplification. Note that larger fragments give brighter signals in the staining procedure due to their higher capacity to bind the DNA dye. Quantification of the amplicons was performed using a Bioanalyzer DNA chip (b). This revealed a strong increase in uniformity of the amplified products when using etPCR compared to standard PCR. Abbreviations: M = Size Marker; P1 = standard PCR; P2 = efficiency tag PCR; C = amplicon concentration after amplification in pmol/l; R = Ratio to lowest abundant target.

The invention is further illustrated by the following working examples:

**EXAMPLE** 1

Oligonucleotide probes are designed to target three genomic locations of the Calpain-3 gene, namely Exon 17, Exon 18&19 and Exon 22, as shown in Fig. 3. For each of the targeted regions, a first oligonucleotide probe ("reverse oligonucleotide") and a second oligonucleotide probe ("forward oligonucleotide") are synthesized. The oligonucleotide probes are given in Table 1 below.

The reverse oligonucleotide probes (CAPN3_Exon17_rev_ET1, CAPN3_Exon18-19_rev_ET5 and CAPN3_Exon22_rev_ET1 for the respective exon) are phosphorylated at the 5' end and comprise a portion of the target sequence complementary to the primal target sequence, the efficiency tag sequence (underlined), the universal reverse primer annealing sequence and six phosphorothioate analogues of nucleotides at their 3' end (indicated by an asterisk).

The forward oligonucleotide probe (CAPN3_Exon17_for_ET1, CAPN3_Exon18-19_for_ET5 and CAPN3_Exon22_for_ET1) comprises six phosphorothioate analogues of nucleotides at their 5' end, a universal forward primer annealing complementary sequence, an efficiency tag complementary sequence (underlined) and a portion of the target sequence complementary to the primal target sequence.

**Table 1**

| Designation | Sequence | SEQ ID NO: |
|---|---|---|
| CAPN3_Exon17_for_ET1 | G*T*A*C*T*A*_CTACACGACGCTCTTCCGATCTTAA_CAGAGGAGCTTGCCTCACA | 1 |
| CAPN3_Exon18-19_for_ET5 | G*T*A*C*T*A*_CTACACGACGCTCTTCCGATCGCTC_TTTGTTTTGCAAAGTGTCCG | 2 |
| CAPN3_Exon22_for_ET1 | G*T*A*C*T*A*_CTACACGACGCTCTTCCGATCTTAA_AGGGAAAATAGAGGCAGGC | 3 |
| CAPN3_Exon17_rev_ET5 | [Phos]- GGTGCCCAGTCAGGCAAAG_CTGG__TCGTATGCCGTCTTCTGCTTG_*G*T*A*C*T*A | 4 |
| CAPN3_Exon18-19_rev_ET5 | [Phos]- GGTGCCCAGTCAGGCAAAG_CTGG__TCGTATGCCGTCTTCTGCTTG_*G*T*A*C*T*A | 5 |
| CAPN3_Exon22_rev_ET5 | [Phos]- GCAACAGGCATCTCACCTGA_CTGG__TCGTATGCCGTCTTCTGCTTG_*G*T*A*C*T*A | 6 |

To hybridize the oligonucleotide probes to genomic DNA, a 10 μl reaction containing 200 pM oligonucleotide probes and 1 μg genomic DNA in 1x amplication buffer (Epicentre) is incubated at 95°C for 5 min, cooled down to 60°C in a PCR cycler using a ramp rate of 1°C per minute. After 14 hours hybridization at 60°C two units Stoffel Polymerase (Applied Biosystems), 10 units Ampligase (Epicentre) and dNTPS with a final concentration of 12 μM are added and incubated at 60°C for 2 more hours. After the gap filling reaction the samples are digested using a exonuclease mix (Exonuclease I, Exonuclease III, Exonuclease Lambda) for 2 hours at 37°C. After heat inactivation of the exonuclease at 80°C for 20min, 1μl of the resulting sample is used for uniform amplification using etPCR.

For uniform amplification using etPCR, a set of primer oligonucleotides comprising a universal primer sequence is used, as given in Table 2.

**Table 2**

| designation | sequence | SEQ ID NO: |
|---|---|---|
| (UFP1): | CTA CAC GAC GCT CTT CCG ATC | 7 |

(continued)

| designation | sequence | SEQ ID NO: |
|---|---|---|
| (UFP2): | CTA CAC GAC GCT CTT CCG ATC G | 8 |
| (UFP3): | CTA CAC GAC GCT CTT CCG ATC GC | 9 |
| (UFP4): | CTA CAC GAC GCT CTT CCG ATC GCT | 10 |
| (UFP5): | CTA CAC GAC GCT CTT CCG ATC GCT C | 11 |
| (URP1): | CAA GCA GAA GAC GGC ATA CGA | 12 |

As primer oligonucleotides a 7:1:1:1:1 mixture of the forward primer oligonucleotides UFP1 (7 parts), UFP2 (1 part), UFP3 (1 part), UFP4 (1 part), UFP5 (1 part) is used at a concentration of 200 nM total forward primer oligonucleotides and 200 nM of universal reverse primer oligonucleotide 1 (URP1). PCR amplification is done using Power SYBR Green Master Mix (Applied Biosystems) and a StepOnePlus Thermocycler with the following PCR program: initial denaturation for 15 minutes at 95°C followed by 40 amplification cycles (10 sec at 95°C, 15 sec at 60°C, 30 sec at 72°C). Amplified targets are analyzed on a 1% agarose gel.

As depicted in Fig. 4, the agarose gel shows that by the method of the present invention a more uniform abundance of replicates are achieved than with standard PCR, which hardly shows any amplification of the Exon 18&19.

Although the specific working example refers to a method in which only on one end an ETS is provided for which five different primer oligonucleotides are used for the polymerase mediated extension, it is understood that an ETS and a set of different oligonucleotides may additionally be used for the opposite end. If also at the opposite end an ETS of four nucleotides and correspondingly a set comprising five different primer oligonucleotides are used, 25 different efficiency grades of amplification may be obtained.

## EXAMPLE 2

### Results

As a model we selected the human dystrophin gene, which is the largest (not exon wise but coverage wise) known human gene consisting of 79 exons. Since the first report of multiplex PCR by Chamberlain the dystrophin gene has been used as a model for multiplex PCR also by other investigators. To establish our new technology we designed 78 different targets covering all 79 exons by using ExonPrimer. To allow fast analyis by gel electrophoresis we selected 12 targets which differ in size to be easily discriminated when resolved on a gel (Figure 5). The sizes of the selected targets are ranging between 153 bp and 725 bp (Figure 5).

To prove the ability of etPCT to control PCR efficiency we first generated single templates with efficiency tags and the common priming sequence by PCR for each of the 12 targets (Figure 5). The gel purified templates were subjected to quantitive PCR to analyze PCR efficiency (Figure 6a). We first used standard qPCR by using a universe primer pair. As all the templates have the same primer binding site and qPCR were performed with the same conditions differences in PCR efficiencies were expected to be due to the intrinsic template properties, like length, GC content and secondary structures. The intrinsic PCR Efficiency of the 12 targets ranged from 76% to 87% (100% corresponding to a duplication in one PCR cycle) (Figure 6b). We analyzed the influence of amplicon length and GC content on PCR amplification efficiency. As expected we found a correlation between size and PCR efficiency (Figure 6c). In contrast, no strong correlation was found between efficiency and the GC content in the samples analyzed (Figure 6d). The GC contents below 20% or above 80% are reported to strongly influence PCR efficiency. The GC content of the twelve templates in our study ranged between 34% and 51 % explaining the only minor impact on PCR performance in our study.

We than investigated whether etPCR was able to influence the PCR efficiency of the same templates by using the set of five universal forward primers, just differing in length. The targets had different efficiency tags, and as expected, a tag matching the entire set of universal primers (TAG 5) had no influence on efficiency when performing etPCR (Figure 7a). However targets having efficiency tags with mismatches are amplified significantly different with etPCR than with normal PCR (Figure 7b,c). To investigate if a distinct tag would influence the PCR efficiency of different targets in the same quantitative manner we calculated a correction factor. The correction factor is the ratio between the efficiencies of etPCR and normal PCR of the same template. This correction factor strongly correlates with the type of tag and is independent of the intrinsic nature of the template (Figures 7d). This allows therefore the adjustment of PCR efficiency of each single target specifically.

We wondered whether these efficiency tags could be used to adjust the amplification efficiencies in multiplex reaction to obtain uniform amplification. Targeting oligonucleotides were designed and the capture reaction was performed as described in material and methods. The selection of the efficiency tags were made according the amplicon size to correct for size dependent amplification bias. Using our novel capture technology we were able to capture the targets from small amounts of genomic DNA (200 ng) and successfully amplify them by PCR (Figure 8a). Using conventional PCR for amplification, a strong bias was observed as we expected from our previous PCR efficiency measurements of the single templates. Small amplicons were highly overrepresented and the largest amplicons were hardly detectable by conventional gel electrophoresis. When using etPCR this bias was strongly corrected for each specific target. The amplified products were quantified using the bioanlyzer 2100 DNA chip technology (Figure 8b). The quantification data revealed a strong correction of the amplification bias, leading to quasi uniform amplification (Figure 8d). This demonstrates the power of the novel etPCR technology in amplifying multiple templates in a uniform manner which is the basis to allow sequence analysis in a very cost effective way.

**Discussion**

We have developed efficiency tag PCR (etPCR), a novel method for multiplex PCR that is capable of uniformly amplifying multiple targets from genomic DNA simultaneously. The target selection protocol is an addition-only reaction and can be performed in a single tube per sample, making it amenable to automation. The application of etPCR is manifold: molecular diagnostics for genetic testing, prenatal testing, cancer profiling as well as for diagnosis of infectious disease organisms and their resistances. In addition it can be applied in forensic applications, detection of genetically modified organisms (GMO) in food and feed, environmental and water testing or synthetic biology.

In this study we focused on the application of etPCR in molecular diagnosis for inherented disorders like Duchene Muscular Dystrophy. The etPCR can be performed on multiple samples in parallel, which can then be labeled with sample-specific DNA barcodes and sequenced as a pool. The choice of targets and target boundaries is flexible, and a wide range of target sequences can be amplified simultaneously (here, 154 bp to 724 bp). Based on the obtained results further adjustment of the efficiency tag can be made, thereby improving uniformity. The number of cycles of adjustment that have to be performed to obtain best uniformity has to be evaluated.

Recently, several new methods have been developed for the multiplex selection, amplification, and sequencing of genomics subsets (Fredriksson et al. 2007, Bashiardes et al. 2005, Dahl et al. 2005, Dahl et al. 2007, Albert et al. 2007, Hodges et al. 2007, Meuzelaar et al. 2007, Okou et al. 2007, Porreca et al. 2007). Several of these methods have several performance disadvantages in different areas, such as the precise definition of target boundaries (Albert et al. 2007, Dahl et al. 2007, Dahl et al. 2005, Okou et al. 2007), the reproducible capture of target regions (Porreca et al. 2007), or the fraction of reads matching target sequences (Albert et al. 2007, Hodges et al. 2007, Okou et al. 2007). In this proof-of-principle study, we did not determine the upper limit of the number of target sequences that can be amplified by etPCR, making it difficult to directly compare our method to these technologies, particularly for applications where a high degree of multiplexing is required. However, etPCR should prove useful for the amplification of an intermediate number (10-1000) of candidate regions in a large number of samples. It is particularly well suited for these applications because it can incorporate sample-specific DNA barcodes, allowing for the precise definition of the boundaries of targeted sequences, is reproducible, is highly specific, and uniformly amplifies the targeted sequences.

We anticipate that etPCR will be useful for a variety of applications. Because the method is based on PCR, it will likely have the same sensitivity as PCR to detect pathogen DNA in a high background of host DNA (Elnifro et al. 2000; Akhras et al. 2007a, b) or to detect rare DNA biomarkers in samples (Fackler et al. 2006). Also, it is likely to have the sensitivity to amplify targets from degraded samples, an area for which there are no robust methods to allow for multiplexed or genome-wide amplification. Other applications that rely heavily on PCR may benefit from higher levels of multiplexing, such as the engineered assembly of many DNA fragments simultaneously in synthetic biology experiments (Reisinger et al. 2006; Forster and Church 2007). By barcoding different samples this method will be useful for selectively sequencing candidate regions in large cohorts of patients to identify variants associated with disease. EtPCR promises to improve many other methods that rely on the sensitivity of PCR and could benefit from higher multiplexing and uniformity such as pathogen detection, biomarker detection in body fluids, and for synthetic DNA assembly.

**Materials and Methods**

Oligonucleotide Design

To design primer for targeting the 79 exons of the dystrophin gene we extracted the genomic sequence information from the GRCh37/hg19 build using the UCSC Genome Browser. Templates including target specific sequences for the target oligonucleotides were selected using the ExonPrimer software, which is based on the Primer3 algorithm. To facilitate fast analysis by gel electrophoresis we selected the 12 templates with a highest diversity concerning target size. To target specific genomic regions a left target oligonucleotide (LTO) and a right target oligonucleotide (RTO) are designed for each selected target. The LTO is capped at the 5 prime end by phosphotioate nucleotides functioning as an exonuclease block. The block is followed by a universal sequence common to all targets to allow PCR amplification and by an efficiency tag necessary to control uniform amplification. Finally, the 3 prime end is composed of a target specific sequence. The RTO is composed contrariwise, starting with the target specific sequence at the 5 prime end and ending with an exonuclease block at the 3 prime end. Additionally the RTO are 5 prime phosphorylated. Oligonucleotides were synthesized by Microsynth (Switzerland), pooled in groups with similar length and gel purified.

Target Oligonucleotide Sequences

**Table 3**

| Designation | Sequence | SEQ ID NO: |
|---|---|---|
| Exon1_L | C*G*T*A*TCGCCTCCCTCGCGCCATCAG<u>GCAA</u>TGCTTCTTTGCAAACTACTGTGAT | 13 |
| Exon3_L | C*G*T*A*TCGCCTCCCTCGCGCCATCAG<u>GCTA</u>TGCTGTTTCAATCAGTACCTAGTCA | 14 |
| Exon7_L | C*G*T*A*TCGCCTCCCTCGCGCCATCAG<u>GCTC</u>CCATCCATAGGGCATACACA | 15 |
| Exon23_L | C*G*T*A*TCGCCTCCCTCGCGCCATCAG<u>GCTC</u>AAGATGCTGAAGGTCAAATGC | 16 |
| Exon19_L | C*G*T*A*TCGCCTCCCTCGCGCCATCAG<u>GCTC</u>TGAACTCAAAGTTGAATTTCTCC | 17 |
| Exon26_L | C*G*T*A*TCGCCTCCCTCGCGCCATCAG<u>GCTA</u>CAACTTCAAGCATTGTTGCAT | 18 |
| Exon32_L | C*G*T*A*TCGCCTCCCTCGCGCCATCAG<u>TTAA</u>GCGTATTTGCCACCAGAAAT | 19 |
| Exon43_L | C*G*T*A*TCGCCTCCCTCGCGCCATCAG<u>GCTA</u>TTTTCCATGGAGGGTACTGA | 20 |
| Exon46_L | C*G*T*A*TCGCCTCCCTCGCGCCATCAG<u>GCTA</u>GGCAGAAAACCAATGATTGAA | 21 |
| Exon59_L | C*G*T*A*TCGCCTCCCTCGCGCCATCAG<u>GCTC</u>TTGTGGGAAGATAACACTGCAC | 22 |
| Exon73_L | C*G*T*A*TCGCCTCCCTCGCGCCATCAG<u>GCAA</u>GCTATCCTACCTCTAAATCCCTCA | 23 |
| Exon79_L | C*G*T*A*TCGCCTCCCTCGCGCCATCAG<u>GCAA</u>TCTGCTCCTTCTTCATCTGTCA | 24 |
| Exon1_R | [Phos]-GAAACCAACAAACTTCAGCAGCTTGGCTGAGCGGGCTGGCAAGGCGC*A*T*A*G | 25 |
| Exon3_R | [Phos]-CACGATTATCCCCTTTTGAAAACTTATTCTGAGCGGGCTGGCAAGGCGC*A*T*A*G | 26 |
| Exon7_R | [Phos]-CTCATTGGGTGTGGTGGCTCTAGGCTGAGCGGGCTGGCAAGGCGC*A*T*A*G | 27 |
| Exon23_R | [Phos]-GCATTTGTGATACAGTTAATGGAGTTGTTGGCTGAGCGGGCTGGCAAGGCGC*A*T*A*G | 28 |
| Exon19_R | [Phos]-AACATCAAAATGGCAATAAAAGCATATTCTGAGCGGGCTGGCAAGGCGC*A*T*A*G | 29 |
| Exon26_R | [Phos]-AAAATAACTCATGGGGATCAGATACATTGGCTGAGCGGGCTGGCAAGGCGC*A*T*A*G | 30 |
| Exon32_R | [Phos]-TTTCCAATGCAGGCAAGTGCTTGGCTGAGCGGGCTGGCAAGGCGC*A*T*A*G | 31 |
| Exon43_R | [Phos]-TCCCAAAGGTAGCAAATGGTGTAGGCTGAGCGGGCTGGCAAGGCGC*A*T*A*G | 32 |
| Exon46_R | [Phos]-CTGGGACACAAACATGGCAATATGCTGAGCGGGCTGGCAAGGCGC*A*T*A*G | 33 |
| Exon59_R | [Phos]-TTGGCATAAATTTTGATACAGCCCTATGCTGAGCGGGCTGGCAAGGCGC*A*T*A*G | 34 |
| Exon73_R | [Phos]-TTCAAGACCTAATCGAACATTCCTGTAGGCTGAGCGGGCTGGCAAGGCGC*A*T*A*G | 35 |
| Exon79_R | [Phos]-GCCATTTGGGAAATCATTCCCTATTCTGAGCGGGCTGGCAAGGCGC*A*T*A*G | 36 |

PCR Amplification of single templates

To analyze the effect of efficiency tags on single targets, templates were produced by standard PCR with the LTOs described above and "right" PCR Primers, which were complementary to the right target oligonucleotides without phosphorylation. Amplification was done with 100 ng genomic DNA, 200 nM of each primers and a commercially available Mastermix (SolisBiodyne) containing a hot start Taq Polymerase and 2.5 mM MgCl. PCR was performed according the following cycling protocol: 95°C for 12min, 35 cycles with 20 seconds for 95 °C, 20 seconds 60°C, 1 minute 72 °C, and a final extension step of 5 minutes at 72°C. PCR products were gel purified and quantified.

**Table 4**

| | | |
|---|---|---|
| Exon1_P | CTATGCGCCTTGCCAGCCCGCTCAGCCAAGCTGCTGAAGTTTGTTGGTTTC | 37 |
| Exon3_P | CTATGCGCCTTGCCAGCCCGCTCAGAATAAGTTTTCAAAAGGGGATAATCGTG | 38 |
| Exon7_P | CTATGCGCCTTGCCAGCCCGCTCAGCCTAGAGCCACCACACCCAATGAG | 39 |
| Exon23_P | CTATGCGCCTTGCCAGCCCGCTCAGCCAACAACTCCATTAACTGTATCACAAATGC | 40 |
| Exon19_P | CTATGCGCCTTGCCAGCCCGCTCAGAATATGCTTTTATTGCCATTTTGATGTT | 41 |
| Exon26_P | CTATGCGCCTTGCCAGCCCGCTCAGCCAATGTATCTGATCCCCATGAGTTATTTT | 42 |
| Exon32_P | CTATGCGCCTTGCCAGCCCGCTCAGCCAAGCACTTGCCTGCATTGGAAA | 43 |
| Exon43_P | CTATGCGCCTTGCCAGCCCGCTCAGCCTACACCATTTGCTACCTTTGGGA | 44 |
| Exon46_P | CTATGCGCCTTGCCAGCCCGCTCAGCATATTGCCATGTTTGTGTCCCAG | 45 |
| Exon59_P | CTATGCGCCTTGCCAGCCCGCTCAGCATAGGGCTGTATCAAAATTTATGCCAA | 46 |
| Exon73_P | CTATGCGCCTTGCCAGCCCGCTCAGCCTACAGGAATGTTCGATTAGGTCTTGAA | 47 |
| Exon79_P | CTATGCGCCTTGCCAGCCCGCTCAGAATAGGGAATGATTTCCCAAATGGC | 48 |

**Table 5**

| | | |
|---|---|---|
| F1 | CGTATCGCCTCCCTCGCGCCATCAG | 49 |
| F2 | CGTATCGCCTCCCTCGCGCCATCAGG | 50 |
| F3 | CGTATCGCCTCCCTCGCGCCATCAGGC | 51 |
| F4 | CGTATCGCCTCCCTCGCGCCATCAGGCT | 52 |
| F5 | CGTATCGCCTCCCTCGCGCCATCAGGCTC | 53 |
| R1 | CTATGCGCCTTGCCAGCCCGCTCAG | 54 |
| R2 | CTATGCGCCTTGCCAGCCCGCTCAGC | 55 |
| R3 | CTATGCGCCTTGCCAGCCCGCTCAGCC | 56 |
| R4 | CTATGCGCCTTGCCAGCCCGCTCAGCCA | 57 |
| R5 | CTATGCGCCTTGCCAGCCCGCTCAGCCAG | 58 |

Quantitative PCR

Quantitative PCR (qPCR) was performed using the StepOnePlus Cylcer (Applied Biosystems) and Power SYBR Green PCR Master Mix (Invitrogen). Primer concentration in all experiments was 200 nM and template concentrations were 10 attomole and 3.3 attomole. Fourty Cylces were performed with following steps: denaturation for 20" at 95 °C, annealing for 20" at 60°C and elongation at 72 °C for 60". Individual PCR efficiencies were calculated by a linear regression analysis using the software package LinReg.

Sequence Capture Reaction

To enrich the selected targets a 10 $\mu$l capture reaction was established using following components: 1 fmol of each target oligonucleotide probes, 200 ng genomic DNA, 0.5 U Phusion Hot Start Polymerase, 5 U Ampligase, 0.1 mM dNTPs in 1x ampligase buffer (Epicentre). The reaction was performed in a PCR cycler with following steps: 1) 95°C for 5 min, 56°C for 2 h, and finally hold at 4°C. After the initial gap filling reaction 5 $\mu$l of an exonuclease cocktail (Exonuclease I, Exonuclease III, Exonuclease lambda) was added. After digestion of the not incorporated oligonucleotide probes as well as the genomic DNA for 1 hour at 37°C the exonucleases were heat inactivated for 10 min at 80°C and the samples were stored at 4°C. Before amplification by etPCR 2 $\mu$l of 50mM EDTA was added. For etPCR amplification 5 $\mu$l of this capture reaction was used.

Multiplex Efficiency Tag PCR

For the etPCR following set of universal primer oligonucleotides were use: a set of forward primer oligonucleotides consisting of F1 (1 part), F2 (2 parts), F3 (3 parts), F4 (4 parts), F5 (5 parts) and R1 as reverse primer oligonucleotide. PCR amplification was done in 30 $\mu$l using 0.2 mM dNTPs, 200 nM of total forward primer oligonucleotides and 200 nM of reverse primer oligonucleotide, 5 ul of capture reaction, 1x GC Phusion buffer, 0.3 $\mu$l Phusion Hot Start polymerase, 2.5 mM MgCl2. The amplification reaction was performed in a Thermocylcer using following cycling program: initial denaturation for 15 minutes at 95°C followed by 40 amplification cycles (10 sec at 95°C, 20 sec at 60°C, 45 sec at 72°C). Amplified targets were analyzed on a 1.8% agarose gel and using the bioanalyszer 2100 system from agilent.

**References**

Albert, T. J., M. N. Molla, D. M. Muzny, L. Nazareth, D. Wheeler, X. Song, T. A. Richmond, C. M. Middle, M. J. Rodesch, C. J. Packard, G. M. Weinstock & R. A. Gibbs (2007) Direct selection of human genomic loci by microarray hybridization. Nat Methods, 4, 903-5.

Bashiardes, S., R. Veile, C. Helms, E. R. Mardis, A. M. Bowcock & M. Lovett (2005) Direct genomic selection. Nat Methods, 2, 63-9.

Broude, N. E., L. Zhang, K. Woodward, D. Englert & C. R. Cantor (2001) Multiplex allele-specific target amplification based on PCR suppression. Proc Natl Acad Sci U S A, 98, 206-11.

Chamberlain, J. S., R. A. Gibbs, J. E. Ranier, P. N. Nguyen & C. T. Caskey (1988) Deletion screening of the Duchenne muscular dystrophy locus via multiplex DNA amplification. Nucleic Acids Res, 16, 11141-56.

Chee, M., R. Yang, E. Hubbell, A. Berno, X. C. Huang, D. Stern, J. Winkler, D. J. Lockhart, M. S. Morris & S. P. Fodor (1996) Accessing genetic information with high-density DNA arrays. Science, 274, 610-4.

Dahl, F., M. Gullberg, J. Stenberg, U. Landegren & M. Nilsson (2005) Multiplex amplification enabled by selective circularization of large sets of genomic DNA fragments. Nucleic Acids Res, 33, e71.

Dahl, F., J. Stenberg, S. Fredriksson, K. Welch, M. Zhang, M. Nilsson, D. Bicknell, W. F. Bodmer, R. W. Davis & H. Ji (2007) Multigene amplification and massively parallel sequencing for cancer mutation discovery. Proc Natl Acad Sci U S A, 104, 9387-92.

Fredriksson, S., J. Banér, F. Dahl, A. Chu, H. Ji, K. Welch & R. W. Davis (2007) Multiplex amplification of all coding sequences within 10 cancer genes by Gene-Collector. Nucleic Acids Research, 35, e47.

Gnirke, A., A. Melnikov, J. Maguire, P. Rogov, E. M. LeProust, W. Brockman, T. Fennell, G. Giannoukos, S. Fisher, C. Russ, S. Gabriel, D. B. Jaffe, E. S. Lander & C. Nusbaum (2009) Solution hybrid selection with ultra-long oligonucleotides for massively parallel targeted sequencing. Nat Biotechnol, 27, 182-9.

Hodges, E., Z. Xuan, V. Balija, M. Kramer, M. N. Molla, S. W. Smith, C. M. Middle, M. J. Rodesch, T. J. Albert, G. J. Hannon & W. R. McCombie (2007) Genome-wide in situ exon capture for selective resequencing. Nat Genet, 39, 1522-7.

Margulies, M., M. Egholm, W. E. Altman, S. Attiya, J. S. Bader, L. A. Bemben, J. Berka, M. S. Braverman, Y. J. Chen, Z. Chen, S. B. Dewell, L. Du, J. M. Fierro, X. V. Gomes, B. C. Godwin, W. He, S. Helgesen, C. H. Ho, G. P. Irzyk, S. C. Jando, M. L. Alenquer, T. P. Jarvie, K. B. Jirage, J. B. Kim, J. R. Knight, J. R. Lanza, J. H. Leamon, S. M. Lefkowitz, M. Lei, J. Li, K. L. Lohman, H. Lu, V. B. Makhijani, K. E. McDade, M. P. McKenna, E. W. Myers, E. Nickerson, J. R. Nobile, R. Plant, B. P. Puc, M. T. Ronan, G. T. Roth, G. J. Sarkis, J. F. Simons, J. W. Simpson, M. Srinivasan, K. R. Tartaro, A. Tomasz, K. A. Vogt, G. A. Volkmer, S. H. Wang, Y. Wang, M. P. Weiner, P. Yu, R. F. Begley & J. M. Rothberg (2005) Genome sequencing in microfabricated high-density picolitre reactors. Nature, 437, 376-80.

Meuzelaar, L. S., O. Lancaster, J. P. Pasche, G. Kopal & A. J. Brookes (2007) MegaPlex PCR: a strategy for multiplex amplification. Nat Methods, 4, 835-7.

Okou, D. T., K. M. Steinberg, C. Middle, D. J. Cutler, T. J. Albert & M. E. Zwick (2007) Microarray-based genomic selection for high-throughput resequencing. Nat Methods, 4, 907-9.

Patil, N., A. J. Berno, D. A. Hinds, W. A. Barrett, J. M. Doshi, C. R. Hacker, C. R. Kautzer, D. H. Lee, C. Marjoribanks, D. P. McDonough, B. T. Nguyen, M. C. Norris, J. B. Sheehan, N. Shen, D. Stern, R. P. Stokowski, D. J. Thomas, M. O. Trulson, K. R. Vyas, K. A. Frazer, S. P. Fodor & D. R. Cox (2001) Blocks of limited haplotype diversity revealed by high-resolution scanning of human chromosome 21. Science, 294, 1719-23.

Porreca, G. J., K. Zhang, J. B. Li, B. Xie, D. Austin, S. L. Vassallo, E. M. LeProust, B. J. Peck, C. J. Emig, F. Dahl, Y. Gao, G. M. Church & J. Shendure (2007) Multiplex amplification of large sets of human exons. Nat Methods, 4, 931-6.

Shendure, J., G. J. Porreca, N. B. Reppas, X. Lin, J. P. McCutcheon, A. M. Rosenbaum, M. D. Wang, K. Zhang, R. D. Mitra & G. M. Church (2005) Accurate multiplex polony sequencing of an evolved bacterial genome. Science, 309, 1728-32.

Shigemori, Y., T. Mikawa, T. Shibata & M. Oishi (2005) Multiplex PCR: use of heat-stable Thermus thermophilus RecA protein to minimize non-specific PCR products. Nucleic Acids Res, 33, e126.

Syvänen, A. C. (2005) Toward genome-wide SNP genotyping. Nat Genet, 37 Suppl, S5-10.

Tewhey, R., J. B. Warner, M. Nakano, B. Libby, M. Medkova, P. H. David, S. K. Kotsopoulos, M. L. Samuels, J. B. Hutchison, J. W. Larson, E. J. Topol, M. P. Weiner, O. Harismendy, J. Olson, D. R. Link & K. A. Frazer (2009) Microdroplet-based PCR enrichment for large-scale targeted sequencing. Nat Biotechnol, 27, 1025-31.

**Claims**

1. A method for the simultaneous amplification of a plurality of different nucleic acid target sequences comprising the steps of
   providing a set of forward primer oligonucleotides capable of annealing to the same nucleotide sequence, comprising a first forward primer oligonucleotide having the structure

5'- X-N$^1$ -3',
and a second forward primer oligonucleotide having the structure
5'- X-N$^1$-N$^2$ -3',
wherein X is a nucleotide sequence which is capable of annealing to a first primer annealing sequence, N$^1$ is nothing or consists of one or more nucleotides, and N$^2$ consists of one or more nucleotides;
providing a plurality of different nucleic acid polymers as templates, each template comprising (i) a forward primer annealing sequence X' which is complementary to the nucleotide sequence X, and (ii) a specific target sequence; and amplifying the templates by a polymerase dependent amplification reaction using said set of forward primer oligonucleotides and one or more reverse primer oligonucleotide(s), **characterized in that** the 3'-terminal nucleotide of the first forward primer oligonucleotide, when annealed to the templates, has a perfect match with at least two different template sequences, and the 3'-terminal nucleotide of the second forward primer oligonucleotide, when annealed to the templates, has a mismatch with at least one of said at least two different template sequences and a perfect match with at least one of said at least two different template sequences.

2. The method of claim 1, further comprising the steps of providing a set of reverse primer oligonucleotides capable of annealing to the same nucleotide sequence, comprising a first reverse primer oligonucleotide having the structure
5'- Y-M$^1$ -3',
and a second reverse primer oligonucleotide having the structure
5'- Y-M$^1$-M$^2$ -3',
wherein Y is a nucleotide sequence which is capable of annealing to a reverse primer annealing sequence, M$^1$ is nothing or consists of one or more nucleotides, and M$^2$ consists of one or more nucleotides;
wherein each template further comprises a reverse primer annealing sequence which is complementary to the nucleotide sequence Y, the target sequence is located between the forward primer annealing sequence and the reverse primer annealing sequence, and the polymerase dependent amplification reaction is carried out using said set of forward primer oligonucleotides and said set of reverse primer oligonucleotides,
**characterized in that** the 3'-terminal nucleotide of the first reverse primer oligonucleotide, when annealed to the templates, has a perfect match with at least two different template sequences, and the 3'-terminal nucleotide of the second reverse primer oligonucleotide, when annealed to the templates, has a mismatch with at least one of said at least two different template sequences and a perfect match with at least one of said at least two different template sequences.

3. The method of claim 2, wherein the number of templates is v, and each template comprises the structure

$$5'\text{-} \ X \text{-} et^{Xw} \text{-} T^w \text{-} et^{Y'w} \text{-} Y' \ \text{-}3'$$

wherein
v is an integer greater than 1,
w is an integer running from 1 to v, each specific template being assigned an individual value w,
X is as defined in claim 1,
$et^{Xw}$ is a first efficiency tag sequence,
$T^w$ is the target sequence or complement thereof,
$et^{Y'w}$ is the complementary sequence of a second efficiency tag sequence,
Y' is the reverse primer annealing sequence.

4. The method of claim 3, **characterized in that** each efficiency tag sequence comprises from 2 to 10 nucleotides, preferably from 2 to 7 nucleotides, most preferably from 3 to 5 nucleotides.

5. The method of claim 3 or 4, **characterized in that** each of the templates is provided by the subsequent steps of:

providing a single stranded primal nucleic acid polymer comprising a primal target sequence to be amplified;
hybridizing to the 5'-end of the primal target sequence an oligonucleotide probe, the sequence of the oligonucleotide probe comprising a portion of the target sequence complementary to the 5'-end of the primal target sequence, the primer annealing sequence and the efficiency tag sequence, and to the 3'-end of the primal target sequence a further oligonucleotide probe, the sequence of the further oligonucleotide probe comprising a portion of the target sequence complementary to the 3'-end of the primal target sequence, the primer annealing complementary sequence and the efficiency tag complementary sequence;

synthesizing a strand complementary to the primal target sequence by means of a polymerase and a ligase to produce the template; and
isolating the templates produced.

6. The method of claim 5, **characterized in that** the ends of the template produced are protected against exonucleases.

7. The method of claim 5 or 6, **characterized in that** the template produced comprises free ends, one or more nucleotides in the region of both ends being modified to form an exonuclease protection.

8. The method of claim 7, **characterized in that** the one or more modified nucleotides are phosphorothioated.

9. The method of any of claims 5 to 8, **characterized in that** the step of isolating the templates produced is performed by digesting the remaining nucleic acid components with an exonuclease.

10. A library of nucleic acid polymers, in particular a DNA or a RNA library, comprising a plurality of templates as defined in any one of claims 2 to 9.

11. A kit for carrying out the method according to any of claims 1 to 9 comprising
a first set of oligonucleotide probes, the sequence of each oligonucleotide probe of the first set comprising

- a portion of a target sequence complementary to the 5'-end of a primal target sequence to be amplified,
- an efficiency tag sequence, and
- a primer annealing sequence,

a second set of oligonucleotide probes, the sequence of each oligonucleotide probe of the second set comprising

- a primer annealing complementary sequence,
- an efficiency tag complementary sequence, and
- a portion of the target sequence complementary to the 3'-end of the primal target sequence,

a first set of different primer oligonucleotides comprising a primer sequence which is at least essentially complementary to the primer annealing sequence of the oligonucleotide of the first set and differing from each other in the length of their extension downstream of the primer sequence, and
a second set of different primer oligonucleotides comprising a primer sequence which is at least essentially complementary to the further primer annealing sequence obtainable by synthesizing a strand complementary to the template comprising the primer annealing complementary sequence, the primer oligonucleotides of the second set differing from each other in the length of their extension downstream of the primer sequence.

12. The kit of claim 11 further comprising a polymerase and a ligase.

13. The use of the method according to any of claims 1 to 9 for a gene probe assay, in particular for identifying infectious organisms or mutant genes.

14. The use of the method according to any of the claims 1 to 9 in molecular cloning.

EP 2 728 013 A1

**Figure 1**

Fig. 1A

Fig. 1B

Fig. 1C

**Figure 2A-C**

**Figure 2D**

**Figures 3 & 4**

Ex17    Ex18_19    Ex22

**Fig. 3**

P1    P2

Ex18_19 →

Ex22 →
Ex17 →

**Fig. 4**

**Figure 5**

**a**

**b**

| Exon | Genomic Target | | Amplicon | | Eff.ID | Eff.Tag A | Eff.Tag B |
|---|---|---|---|---|---|---|---|
| | size (bp) | GC content | size (bp) | GC content | | | |
| Exon 1 | 255 | 33% | 313 | 39% | 14 | GCAA | CCAA |
| Exon 3 | 290 | 37% | 348 | 41% | 16 | GCTA | AATA |
| Exon 7 | 585 | 34% | 643 | 37% | 23 | GCTC | CCTA |
| Exon 19 | 460 | 34% | 518 | 37% | 21 | GCTC | AATA |
| Exon 23 | 666 | 31% | 724 | 34% | 24 | GCTC | CCAA |
| Exon 26 | 411 | 32% | 469 | 36% | 19 | GCTA | CCAA |
| Exon 32 | 96 | 44% | 154 | 51% | 4 | TTAA | CCAA |
| Exon 43 | 364 | 34% | 422 | 39% | 18 | GCTA | CCTA |
| Exon 46 | 325 | 33% | 383 | 38% | 17 | GCTA | CATA |
| Exon 59 | 514 | 46% | 572 | 48% | 22 | GCTC | CATA |
| Exon 73 | 217 | 35% | 275 | 42% | 13 | GCAA | CCTA |
| Exon 79 | 185 | 42% | 243 | 47% | 11 | GCAA | AATA |

**c**

**Figure 6**

**Figure 7**

**a**

**b**

**c**

| Amplicon | standard PCR Efficiency | etPCR Efficiency | p-value | Efficiency Tag | Correction Factor |
|---|---|---|---|---|---|
| Exon1 | 1.81 (0.014) | 1.64 (0.013) | <0.00001 | 3 | 0.91 |
| Exon3 | 1.81 (0.014) | 1.75 (0.010) | 0.000392 | 4 | 0.96 |
| Exon7 | 1.82 (0.007) | 1.78 (0.019) | 0.025343 | 5 | 0.98 |
| Exon19 | 1.78 (0.007) | 1.77 (0.007) | 0.144931 | 5 | 1.00 |
| Exon23 | 1.73 (0.005) | 1.72 (0.003) | 0.014902 | 5 | 0.99 |
| Exon26 | 1.79 (0.004) | 1.72 (0.005) | <0.00001 | 4 | 0.96 |
| Exon32 | 1.85 (0.007) | 1.50 (0.003) | <0.00001 | 1 | 0.81 |
| Exon43 | 1.79 (0.009) | 1.70 (0.005) | 2.06E-05 | 4 | 0.95 |
| Exon46 | 1.80 (0.005) | 1.74 (0.002) | 4.31E-05 | 4 | 0.97 |
| Exon59 | 1.76 (0.005) | 1.76 (0.006) | 0.224424 | 5 | 1.00 |
| Exon73 | 1.84 (0.012) | 1.67 (0.005) | 1.04E-05 | 3 | 0.91 |
| Exon79 | 1.83 (0.013) | 1.66 (0.004) | 3.92E-05 | 3 | 0.91 |

**d**

**Figure 8**

a

b

c

Europäisches
Patentamt

European
Patent Office

Office européen
des brevets

# EUROPEAN SEARCH REPORT

Application Number

EP 12 19 0754

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/050242 A2 (RUBICON GENOMICS INC [US]; MAKAROV VLADIMIR L [US]; SLEPTSOVA IRINA [U) 19 June 2003 (2003-06-19) | 10 | INV. C12Q1/68 |
| A | * page 23, [0110]; figures; claim 73 * | 1-9, 11-14 | |
| X | WO 93/06239 A1 (KEYGENE NV [NL]) 1 April 1993 (1993-04-01) | 10 | |
| A | * claims; figures * | 1-9, 11-14 | |
| X | WO 02/083955 A1 (PE CORP NY [US]) 24 October 2002 (2002-10-24) | 10 | |
| A | * pages 10-11; figure 2; claims * | 1-9, 11-14 | |

TECHNICAL FIELDS
SEARCHED (IPC)

C12Q

The present search report has been drawn up for all claims

| Place of search | Date of completion of the search | Examiner |
|---|---|---|
| Munich | 7 February 2013 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 12 19 0754

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2013

| Patent document cited in search report | | Publication date | Patent family member(s) | | Publication date |
|---|---|---|---|---|---|
| WO 03050242 | A2 | 19-06-2003 | AU | 2002359436 A1 | 23-06-2003 |
| | | | EP | 1451365 A2 | 01-09-2004 |
| | | | JP | 2005535283 A | 24-11-2005 |
| | | | US | 2003143599 A1 | 31-07-2003 |
| | | | US | 2010145037 A1 | 10-06-2010 |
| | | | WO | 03050242 A2 | 19-06-2003 |
| WO 9306239 | A1 | 01-04-1993 | AT | 191510 T | 15-04-2000 |
| | | | AT | 382094 T | 15-01-2008 |
| | | | AU | 672760 B2 | 17-10-1996 |
| | | | CA | 2119557 A1 | 01-04-1993 |
| | | | CZ | 291877 B6 | 18-06-2003 |
| | | | CZ | 298187 B6 | 18-07-2007 |
| | | | DE | 69230873 D1 | 11-05-2000 |
| | | | DE | 69230873 T2 | 09-11-2000 |
| | | | DE | 69233719 T2 | 02-01-2009 |
| | | | DK | 0534858 T3 | 11-09-2000 |
| | | | DK | 0969102 T3 | 13-05-2008 |
| | | | EP | 0534858 A1 | 31-03-1993 |
| | | | EP | 0969102 A2 | 05-01-2000 |
| | | | ES | 2147550 T3 | 16-09-2000 |
| | | | ES | 2299229 T3 | 16-05-2008 |
| | | | FI | 941360 A | 24-05-1994 |
| | | | FI | 20031526 A | 17-10-2003 |
| | | | GR | 3033895 T3 | 30-11-2000 |
| | | | HU | 223760 B1 | 28-01-2005 |
| | | | IL | 103267 A | 25-07-2004 |
| | | | JP | 3236295 B2 | 10-12-2001 |
| | | | JP | 4088403 B2 | 21-05-2008 |
| | | | JP | 4385072 B2 | 16-12-2009 |
| | | | JP | H06510668 A | 01-12-1994 |
| | | | JP | 2001061486 A | 13-03-2001 |
| | | | JP | 2008131947 A | 12-06-2008 |
| | | | NO | 941064 A | 20-05-1994 |
| | | | PT | 534858 E | 29-09-2000 |
| | | | PT | 969102 E | 25-03-2008 |
| | | | RU | 2182176 C2 | 10-05-2002 |
| | | | US | 6045994 A | 04-04-2000 |
| | | | WO | 9306239 A1 | 01-04-1993 |
| | | | ZA | 9207323 A | 30-08-1993 |
| WO 02083955 | A1 | 24-10-2002 | CA | 2443999 A1 | 24-10-2002 |
| | | | EP | 1386002 A1 | 04-02-2004 |
| | | | JP | 2004526453 A | 02-09-2004 |
| | | | US | 2003082572 A1 | 01-05-2003 |
| | | | WO | 02083955 A1 | 24-10-2002 |

For more details about this annex : see Official Journal of the European Patent Office, No. 12/82

**ANNEX TO THE EUROPEAN SEARCH REPORT
ON EUROPEAN PATENT APPLICATION NO.**                     EP 12 19 0754

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2013

| Patent document cited in search report | Publication date | Patent family member(s) | Publication date |
|---|---|---|---|
| | | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683202 B


**Non-patent literature cited in the description**

- **ALBERT, T. J. ; M. N. MOLLA ; D. M. MUZNY ; L. NAZARETH ; D. WHEELER ; X. SONG ; T. A. RICHMOND ; C. M. MIDDLE ; M. J. RODESCH ; C. J. PACKARD.** Direct selection of human genomic loci by microarray hybridization. *Nat Methods,* 2007, vol. 4, 903-5
- **BASHIARDES, S. ; R. VEILE ; C. HELMS ; E. R. MARDIS ; A. M. BOWCOCK ; M. LOVETT.** Direct genomic selection. *Nat Methods,* 2005, vol. 2, 63-9
- **BROUDE, N. E. ; L. ZHANG ; K. WOODWARD ; D. ENGLERT ; C. R. CANTOR.** Multiplex allele-specific target amplification based on PCR suppression. *Proc Natl Acad Sci U S A,* 2001, vol. 98, 206-11
- **CHAMBERLAIN, J. S. ; R. A. GIBBS ; J. E. RANIER ; P. N. NGUYEN ; C. T. CASKEY.** Deletion screening of the Duchenne muscular dystrophy locus via multiplex DNA amplification. *Nucleic Acids Res,* 1988, vol. 16, 11141-56
- **CHEE, M. ; R. YANG ; E. HUBBELL ; A. BERNO ; X. C. HUANG ; D. STERN ; J. WINKLER ; D. J. LOCKHART ; M. S. MORRIS ; S. P. FODOR.** Accessing genetic information with high-density DNA arrays. *Science,* 1996, vol. 274, 610-4
- **DAHL, F. ; M. GULLBERG ; J. STENBERG ; U. LANDEGREN ; M. NILSSON.** Multiplex amplification enabled by selective circularization of large sets of genomic DNA fragments. *Nucleic Acids Res,* 2005, vol. 33, e71
- **DAHL, F. ; J. STENBERG ; S. FREDRIKSSON ; K. WELCH ; M. ZHANG ; M. NILSSON ; D. BICKNELL ; W. F. BODMER ; R. W. DAVIS ; H. JI.** Multigene amplification and massively parallel sequencing for cancer mutation discovery. *Proc Natl Acad Sci U S A,* 2007, vol. 104, 9387-92
- **FREDRIKSSON, S. ; J. BANÉR ; F. DAHL ; A. CHU ; H. JI ; K. WELCH ; R. W. DAVIS.** Multiplex amplification of all coding sequences within 10 cancer genes by Gene-Collector. *Nucleic Acids Research,* 2007, vol. 35, e47

- **GNIRKE, A. ; A. MELNIKOV ; J. MAGUIRE ; P. ROGOV ; E. M. LEPROUST ; W. BROCKMAN ; T. FENNELL ; G. GIANNOUKOS ; S. FISHER ; C. RUSS.** Solution hybrid selection with ultra-long oligonucleotides for massively parallel targeted sequencing. *Nat Biotechnol,* 2009, vol. 27, 182-9
- **HODGES, E. ; Z. XUAN ; V. BALIJA ; M. KRAMER ; M. N. MOLLA ; S. W. SMITH ; C. M. MIDDLE ; M. J. RODESCH ; T. J. ALBERT ; G. J. HANNON.** Genome-wide in situ exon capture for selective resequencing. *Nat Genet,* 2007, vol. 39, 1522-7
- **MARGULIES, M. ; M. EGHOLM ; W. E. ALTMAN ; S. ATTIYA ; J. S. BADER ; L. A. BEMBEN ; J. BERKA ; M. S. BRAVERMAN ; Y. J. CHEN ; Z. CHEN.** Genome sequencing in microfabricated high-density picolitre reactors. *Nature,* 2005, vol. 437, 376-80
- **MEUZELAAR, L. S. ; O. LANCASTER ; J. P. PASCHE ; G. KOPAL ; A. J. BROOKES.** MegaPlex PCR: a strategy for multiplex amplification. *Nat Methods,* 2007, vol. 4, 835-7
- **OKOU, D. T. ; K. M. STEINBERG ; C. MIDDLE ; D. J. CUTLER ; T. J. ALBERT ; M. E. ZWICK.** Microarray-based genomic selection for high-throughput resequencing. *Nat Methods,* 2007, vol. 4, 907-9
- **PATIL, N. ; A. J. BERNO ; D. A. HINDS ; W. A. BARRETT ; J. M. DOSHI ; C. R. HACKER ; C. R. KAUTZER ; D. H. LEE ; C. MARJORIBANKS ; D. P. MCDONOUGH.** Blocks of limited haplotype diversity revealed by high-resolution scanning of human chromosome 21. *Science,* 2001, vol. 294, 1719-23
- **PORRECA, G. J ; K. ZHANG ; J. B. LI ; B. XIE ; D. AUSTIN ; S. L. VASSALLO ; E. M. LEPROUST ; B. J. PECK ; C. J. EMIG ; F. DAHL.** Multiplex amplification of large sets of human exons. *Nat Methods,* 2007, vol. 4, 931-6
- **SHENDURE, J. ; G. J. PORRECA ; N. B. REPPAS ; X. LIN ; J. P. MCCUTCHEON ; A. M. ROSENBAUM ; M. D. WANG ; K. ZHANG ; R. D. MITRA ; G. M. CHURCH.** Accurate multiplex polony sequencing of an evolved bacterial genome. *Science,* 2005, vol. 309, 1728-32

- **SHIGEMORI, Y. ; T. MIKAWA ; T. SHIBATA ; M. OI-SHI.** Multiplex PCR: use of heat-stable Thermus thermophilus RecA protein to minimize non-specific PCR products. *Nucleic Acids Res,* 2005, vol. 33, e126
- **SYVÄNEN, A. C.** Toward genome-wide SNP genotyping. *Nat Genet,* 2005, vol. 37, 5-10

- **TEWHEY, R. ; J. B. WARNER ; M. NAKANO ; B. LIBBY ; M. MEDKOVA ; P. H. DAVID ; S. K. KOTSOPOULOS ; M. L. SAMUELS ; J. B. HUTCHISON ; J. W. LARSON.** Microdroplet-based PCR enrichment for large-scale targeted sequencing. *Nat Biotechnol,* 2009, vol. 27, 1025-31

SEQUENCE LISTING

<110> University Hospital Basel

<120> Method for the simultaneous amplification of a plurality of
      different nucleic acid target sequences

<130> BLM0074ZZZ

<160> 58

<170> PatentIn version 3.5

<210> 1
<211> 50
<212> DNA
<213> Artificial Sequence

<220>
<223> CAPN3_Exon17_for_ET1


<220>
<221> misc_feature
<222> (1)..(7)
<223> phosphorothioate internucleotide linkages

<400> 1
gtactactac acgacgctct tccgatctta acagaggagc ttgcctcaca                50


<210> 2
<211> 51
<212> DNA
<213> Artificial Sequence

<220>
<223> CAPN3_Exon18-19_for_ET5


<220>
<221> misc_feature
<222> (1)..(7)
<223> phosphorothioate internucleotide linkages

<400> 2
gtactactac acgacgctct tccgatcgct ctttgttttg caaagtgtcc g              51


<210> 3
<211> 50
<212> DNA
<213> Artificial Sequence

<220>
<223> CAPN3_Exon22_for_ET1


<220>
<221> misc_feature
<222> (1)..(7)
<223> phosphorothioate internucleotide linkages

<400> 3
gtactactac acgacgctct tccgatctta aagggaaaat agaggcaggc                50

```
<210>  4
<211>  50
<212>  DNA
<213>  Artificial Sequence

<220>
<223>  CAPN3_Exon17_rev_ET5


<220>
<221>  misc_feature
<222>  (1)..(1)
<223>  5' phosphorylation

<220>
<221>  misc_feature
<222>  (44)..(50)
<223>  phosphorothioate internucleotide linkages

<400>  4
ggtgcccagt caggcaaagc tggtcgtatg ccgtcttctg cttggtacta          50


<210>  5
<211>  50
<212>  DNA
<213>  Artificial Sequence

<220>
<223>  CAPN3_Exon18-19_rev_ET5


<220>
<221>  misc_feature
<222>  (1)..(1)
<223>  5' phosphorylation

<220>
<221>  misc_feature
<222>  (44)..(50)
<223>  phosphorothioate internucleotide linkages

<400>  5
ggtgcccagt caggcaaagc tggtcgtatg ccgtcttctg cttggtacta          50


<210>  6
<211>  51
<212>  DNA
<213>  Artificial Sequence

<220>
<223>  CAPN3_Exon22_rev_ET5


<220>
<221>  misc_feature
<222>  (1)..(1)
<223>  5' phosphorylation

<220>
<221>  misc_feature
<222>  (45)..(51)
```

<223> phosphorothioate internucleotide linkages

<400> 6
gcaacaggca tctcacctga ctggtcgtat gccgtcttct gcttggtact a    51

<210> 7
<211> 21
<212> DNA
<213> Artificial Sequence

<220>
<223> (UFP1):

<400> 7
ctacacgacg ctcttccgat c    21

<210> 8
<211> 22
<212> DNA
<213> Artificial Sequence

<220>
<223> (UFP2):

<400> 8
ctacacgacg ctcttccgat cg    22

<210> 9
<211> 23
<212> DNA
<213> Artificial Sequence

<220>
<223> (UFP3):

<400> 9
ctacacgacg ctcttccgat cgc    23

<210> 10
<211> 24
<212> DNA
<213> Artificial Sequence

<220>
<223> (UFP4):

<400> 10
ctacacgacg ctcttccgat cgct    24

<210> 11
<211> 25
<212> DNA
<213> Artificial Sequence

<220>
<223> (UFP5):

<400> 11
ctacacgacg ctcttccgat cgctc    25

```
<210>  12
<211>  21
<212>  DNA
<213>  Artificial Sequence

<220>
<223>  URP1

<400>  12
caagcagaag acggcatacg a                                              21


<210>  13
<211>  53
<212>  DNA
<213>  Artificial Sequence

<220>
<223>  Exon1_L


<220>
<221>  misc_feature
<222>  (1)..(5)
<223>  phosphorothioate internucleotide linkages

<400>  13
cgtatcgcct ccctcgcgcc atcaggcaat gcttctttgc aaactactgt gat          53


<210>  14
<211>  54
<212>  DNA
<213>  Artificial Sequence

<220>
<223>  Exon3_L


<220>
<221>  misc_feature
<222>  (1)..(5)
<223>  phosphorothioate internucleotide linkages

<400>  14
cgtatcgcct ccctcgcgcc atcaggctat gctgtttcaa tcagtaccta gtca         54


<210>  15
<211>  49
<212>  DNA
<213>  Artificial Sequence

<220>
<223>  Exon7_L


<220>
<221>  misc_feature
<222>  (1)..(5)
<223>  phosphorothioate internucleotide linkages

<400>  15
cgtatcgcct ccctcgcgcc atcaggctcc catccatagg gcatacaca              49
```

```
<210>  16
<211>  50
<212>  DNA
<213>  Artificial Sequence

<220>
<223>  Exon23_L


<220>
<221>  misc_feature
<222>  (1)..(5)
<223>  phosphorothioate internucleotide linkages

<400>  16
cgtatcgcct ccctcgcgcc atcaggctca agatgctgaa ggtcaaatgc                 50


<210>  17
<211>  52
<212>  DNA
<213>  Artificial Sequence

<220>
<223>  Exon19_L


<220>
<221>  misc_feature
<222>  (1)..(5)
<223>  phosphorothioate internucleotide linkages

<400>  17
cgtatcgcct ccctcgcgcc atcaggctct gaactcaaag ttgaatttct cc             52


<210>  18
<211>  50
<212>  DNA
<213>  Artificial Sequence

<220>
<223>  Exon26_L


<220>
<221>  misc_feature
<222>  (1)..(5)
<223>  phosphorothioate internucleotide linkages

<400>  18
cgtatcgcct ccctcgcgcc atcaggctac aacttcaagc attgttgcat                 50


<210>  19
<211>  49
<212>  DNA
<213>  Artificial Sequence

<220>
<223>  Exon32_L
```

```
<220>
<221>  misc_feature
<222>  (1)..(5)
<223>  phosphorothioate internucleotide linkages

<400>  19
cgtatcgcct ccctcgcgcc atcagttaag cgtatttgcc accagaaat                        49


<210>  20
<211>  49
<212>  DNA
<213>  Artificial Sequence

<220>
<223>  Exon43_L


<220>
<221>  misc_feature
<222>  (1)..(5)
<223>  phosphorothioate internucleotide linkages

<400>  20
cgtatcgcct ccctcgcgcc atcaggctat tttccatgga gggtactga                        49


<210>  21
<211>  50
<212>  DNA
<213>  Artificial Sequence

<220>
<223>  Exon46_L


<220>
<221>  misc_feature
<222>  (1)..(5)
<223>  phosphorothioate internucleotide linkages

<400>  21
cgtatcgcct ccctcgcgcc atcaggctag gcagaaaacc aatgattgaa                       50


<210>  22
<211>  51
<212>  DNA
<213>  Artificial Sequence

<220>
<223>  Exon59_L


<220>
<221>  misc_feature
<222>  (1)..(5)
<223>  phosphorothioate internucleotide linkages

<400>  22
cgtatcgcct ccctcgcgcc atcaggctct tgtgggaaga taacactgca c                     51


<210>  23
<211>  53
```

```
<212>  DNA
<213>  Artificial Sequence

<220>
<223>  Exon73_L


<220>
<221>  misc_feature
<222>  (1)..(5)
<223>  phosphorothioate internucleotide linkages

<400>  23
cgtatcgcct ccctcgcgcc atcaggcaag ctatcctacc tctaaatccc tca                53


<210>  24
<211>  51
<212>  DNA
<213>  Artificial Sequence

<220>
<223>  Exon79_L


<220>
<221>  misc_feature
<222>  (1)..(5)
<223>  phosphorothioate internucleotide linkages

<400>  24
cgtatcgcct ccctcgcgcc atcaggcaat ctgctccttc ttcatctgtc a                  51


<210>  25
<211>  51
<212>  DNA
<213>  Artificial Sequence

<220>
<223>  Exon1_R


<220>
<221>  misc_feature
<222>  (1)..(1)
<223>  5' phosphorylation

<220>
<221>  misc_feature
<222>  (47)..(51)
<223>  phosphorothioate internucleotide linkages

<400>  25
gaaaccaaca aacttcagca gcttggctga gcgggctggc aaggcgcata g                  51


<210>  26
<211>  53
<212>  DNA
<213>  Artificial Sequence

<220>
<223>  Exon3_R
```

```
<220>
<221>  misc_feature
<222>  (1)..(1)
<223>  5' phosphorylation

<220>
<221>  misc_feature
<222>  (49)..(53)
<223>  phosphorothioate internucleotide linkages

<400>  26
cacgattatc cccttttgaa aacttattct gagcgggctg gcaaggcgca tag          53


<210>  27
<211>  49
<212>  DNA
<213>  Artificial Sequence

<220>
<223>  Exon7_R


<220>
<221>  misc_feature
<222>  (1)..(1)
<223>  5' phosphorylation

<220>
<221>  misc_feature
<222>  (45)..(49)
<223>  phosphorothioate internucleotide linkages

<400>  27
ctcattgggt gtggtggctc taggctgagc gggctggcaa ggcgcatag              49


<210>  28
<211>  56
<212>  DNA
<213>  Artificial Sequence

<220>
<223>  Exon23_R


<220>
<221>  misc_feature
<222>  (1)..(1)
<223>  5' phosphorylation

<220>
<221>  misc_feature
<222>  (52)..(56)
<223>  phosphorothioate internucleotide linkages

<400>  28
gcatttgtga tacagttaat ggagttgttg gctgagcggg ctggcaaggc gcatag      56


<210>  29
<211>  53
<212>  DNA
<213>  Artificial Sequence
```

<220>
<223> Exon19_R

<220>
<221> misc_feature
<222> (1)..(1)
<223> 5' phosphorylation

<220>
<221> misc_feature
<222> (49)..(53)
<223> phosphorothioate internucleotide linkages

<400> 29
aacatcaaaa tggcaataaa agcatattct gagcgggctg gcaaggcgca tag          53

<210> 30
<211> 55
<212> DNA
<213> Artificial Sequence

<220>
<223> Exon26_R

<220>
<221> misc_feature
<222> (1)..(1)
<223> 5' phosphorylation

<220>
<221> misc_feature
<222> (51)..(55)
<223> phosphorothioate internucleotide linkages

<400> 30
aaaataactc atggggatca gatacattgg ctgagcgggc tggcaaggcg catag          55

<210> 31
<211> 49
<212> DNA
<213> Artificial Sequence

<220>
<223> Exon32_R

<220>
<221> misc_feature
<222> (1)..(1)
<223> 5' phosphorylation

<220>
<221> misc_feature
<222> (45)..(49)
<223> phosphorothioate internucleotide linkages

<400> 31
tttccaatgc aggcaagtgc ttggctgagc gggctggcaa ggcgcatag          49

```
<210>  32
<211>  50
<212>  DNA
<213>  Artificial Sequence

<220>
<223>  Exon43_R


<220>
<221>  misc_feature
<222>  (1)..(1)
<223>  5' phosphorylation

<220>
<221>  misc_feature
<222>  (46)..(50)
<223>  phosphorothioate internucleotide linkages

<400>  32
tcccaaaggt agcaaatggt gtaggctgag cgggctggca aggcgcatag          50


<210>  33
<211>  49
<212>  DNA
<213>  Artificial Sequence

<220>
<223>  Exon46_R


<220>
<221>  misc_feature
<222>  (1)..(1)
<223>  5' phosphorylation

<220>
<221>  misc_feature
<222>  (45)..(49)
<223>  phosphorothioate internucleotide linkages

<400>  33
ctgggacaca aacatggcaa tatgctgagc gggctggcaa ggcgcatag           49


<210>  34
<211>  53
<212>  DNA
<213>  Artificial Sequence

<220>
<223>  Exon59_R


<220>
<221>  misc_feature
<222>  (1)..(1)
<223>  5' phosphorylation

<220>
<221>  misc_feature
<222>  (49)..(53)
<223>  phosphorothioate internucleotide linkages
```

```
<400>  34
ttggcataaa ttttgataca gccctatgct gagcgggctg gcaaggcgca tag          53


<210>  35
<211>  54
<212>  DNA
<213>  Artificial Sequence

<220>
<223>  Exon73_R


<220>
<221>  misc_feature
<222>  (1)..(1)
<223>  5' phosphorylation

<220>
<221>  misc_feature
<222>  (50)..(54)
<223>  phosphorothioate internucleotide linkages

<400>  35
ttcaagacct aatcgaacat tcctgtaggc tgagcgggct ggcaaggcgc atag         54


<210>  36
<211>  50
<212>  DNA
<213>  Artificial Sequence

<220>
<223>  Exon79_R


<220>
<221>  misc_feature
<222>  (1)..(1)
<223>  5' phosphorylation

<220>
<221>  misc_feature
<222>  (46)..(50)
<223>  phosphorothioate internucleotide linkages

<400>  36
gccatttggg aaatcattcc ctattctgag cgggctggca aggcgcatag             50


<210>  37
<211>  51
<212>  DNA
<213>  Artificial Sequence

<220>
<223>  Exon1_P

<400>  37
ctatgcgcct tgccagcccg ctcagccaag ctgctgaagt ttgttggttt c           51


<210>  38
<211>  53
<212>  DNA
```

<213> Artificial Sequence

<220>
<223> Exon3_P

<400> 38
ctatgcgcct tgccagcccg ctcagaataa gttttcaaaa ggggataatc gtg          53

<210> 39
<211> 49
<212> DNA
<213> Artificial Sequence

<220>
<223> Exon7_P

<400> 39
ctatgcgcct tgccagcccg ctcagcctag agccaccaca cccaatgag          49

<210> 40
<211> 56
<212> DNA
<213> Artificial Sequence

<220>
<223> Exon23_P

<400> 40
ctatgcgcct tgccagcccg ctcagccaac aactccatta actgtatcac aaatgc          56

<210> 41
<211> 53
<212> DNA
<213> Artificial Sequence

<220>
<223> Exon19_P

<400> 41
ctatgcgcct tgccagcccg ctcagaatat gcttttattg ccattttgat gtt          53

<210> 42
<211> 55
<212> DNA
<213> Artificial Sequence

<220>
<223> Exon26_P

<400> 42
ctatgcgcct tgccagcccg ctcagccaat gtatctgatc cccatgagtt atttt          55

<210> 43
<211> 49
<212> DNA
<213> Artificial Sequence

<220>
<223> Exon32_P

<400> 43
ctatgcgcct tgccagcccg ctcagccaag cacttgcctg cattggaaa                49


<210> 44
<211> 50
<212> DNA
<213> Artificial Sequence

<220>
<223> Exon43_P

<400> 44
ctatgcgcct tgccagcccg ctcagcctac accatttgct acctttggga                50


<210> 45
<211> 49
<212> DNA
<213> Artificial Sequence

<220>
<223> Exon46_P

<400> 45
ctatgcgcct tgccagcccg ctcagcatat tgccatgttt gtgtcccag                49


<210> 46
<211> 53
<212> DNA
<213> Artificial Sequence

<220>
<223> Exon59_P

<400> 46
ctatgcgcct tgccagcccg ctcagcatag ggctgtatca aaatttatgc caa                53


<210> 47
<211> 54
<212> DNA
<213> Artificial Sequence

<220>
<223> Exon73_P

<400> 47
ctatgcgcct tgccagcccg ctcagcctac aggaatgttc gattaggtct tgaa                54


<210> 48
<211> 50
<212> DNA
<213> Artificial Sequence

<220>
<223> Exon79_P

<400> 48
ctatgcgcct tgccagcccg ctcagaatag ggaatgattt cccaaatggc                50


<210> 49

<211>    25
<212>    DNA
<213>    Artificial Sequence

<220>
<223>    F1

<400>    49
cgtatcgcct ccctcgcgcc atcag                                    25


<210>    50
<211>    26
<212>    DNA
<213>    Artificial Sequence

<220>
<223>    F2

<400>    50
cgtatcgcct ccctcgcgcc atcagg                                   26


<210>    51
<211>    27
<212>    DNA
<213>    Artificial Sequence

<220>
<223>    F3

<400>    51
cgtatcgcct ccctcgcgcc atcaggc                                  27


<210>    52
<211>    28
<212>    DNA
<213>    Artificial Sequence

<220>
<223>    F4

<400>    52
cgtatcgcct ccctcgcgcc atcaggct                                 28


<210>    53
<211>    29
<212>    DNA
<213>    Artificial Sequence

<220>
<223>    F5

<400>    53
cgtatcgcct ccctcgcgcc atcaggctc                                29


<210>    54
<211>    25
<212>    DNA
<213>    Artificial Sequence

<220>

```
<223>  R1

<400>  54
ctatgcgcct tgccagcccg ctcag                                          25


<210>  55
<211>  26
<212>  DNA
<213>  Artificial Sequence

<220>
<223>  R2

<400>  55
ctatgcgcct tgccagcccg ctcagc                                         26


<210>  56
<211>  27
<212>  DNA
<213>  Artificial Sequence

<220>
<223>  R3

<400>  56
ctatgcgcct tgccagcccg ctcagcc                                        27


<210>  57
<211>  28
<212>  DNA
<213>  Artificial Sequence

<220>
<223>  R4

<400>  57
ctatgcgcct tgccagcccg ctcagcca                                       28


<210>  58
<211>  29
<212>  DNA
<213>  Artificial Sequence

<220>
<223>  R5

<400>  58
ctatgcgcct tgccagcccg ctcagccag                                      29
=
```